(19) Europäisches Patentamt
European Patent Office
Office européen des brevets

(11) Veröffentlichungsnummer: **0 208 950**
**A2**

(12) # EUROPÄISCHE PATENTANMELDUNG

(21) Anmeldenummer: 86108494.5

(22) Anmeldetag: 21.06.86

(51) Int. Cl.[4]: **A 61 F 9/00**, A 61 B 17/32

(30) Priorität: 27.06.85 DE 3522998
27.06.85 DE 3522999
27.06.85 DE 3523015

(43) Veröffentlichungstag der Anmeldung: 21.01.87
Patentblatt 87/4

(84) Benannte Vertragsstaaten: AT BE CH DE FR GB IT LI LU NL SE

(71) Anmelder: Gründler, Patrik, Iddastrasse 7, 9098 St. Gallen (CH)

(72) Erfinder: Gründler, Patrik, Iddastrasse 7, 9098 St. Gallen (CH)

(74) Vertreter: Patentanwälte Dipl.-Ing. E. Eisele Dr.-Ing. H. Otten, Seestrasse 42, D-7980 Ravensburg (DE)

(54) **Vorrichtung zur Transplantation der Cornea des menschlichen Auges.**

(57)
Eine Vorrichtung zur Operation an der Cornea des menschlichen Auges wird erfindungsgemäß aus verschiedenen Instrumenten und Hilfsgeräten derart zusammengesetzt, daß die Transplantation eines scheibchenförmigen Teils der Cornea (perforierende Keratoplastik) weitestgehend maschinell-selbsttätig durchgeführt werden kann, wobei mit der Hand ausgeführte chirurgische Manipulationen nahezu entfallen können. An einem dreidimensional verstellbaren Träger sind ein Achseinstellinstrument (17), ein Trepan (18) und ein Transplantator (19) so angebracht, daß diese Instrumente wahlweise mit der Sehachse des Patienten in Übereinstimmung gebracht und auf eine voreinstellbare Höhe verfahren werden können. Vorzugsweise umfaßt der Träger einen Hilfsträger (8) und einen an diesem um eine horizontale Achse (12) schwenkbaren Drehteller (11). Dieser trägt die Instrumente. An dem Hilfsträger (8) sind ein Bulbushaltering (14) höhenverstellbar und ein Manipulator (15) sowie ein Patronenwechsler (16) unbeweglich angebracht. Die Instrumente und Hilfsvorrichtungen werden im einzelnen beschrieben. Die Vorrichtung insgesamt kann mittels einer Steuerkonsole (20) betätigt werden. Die Funktionen und Programmabläufe sind elektrisch gesteuert, speicherbar und werden auf einem Monitor (21) sichtbar gemacht.

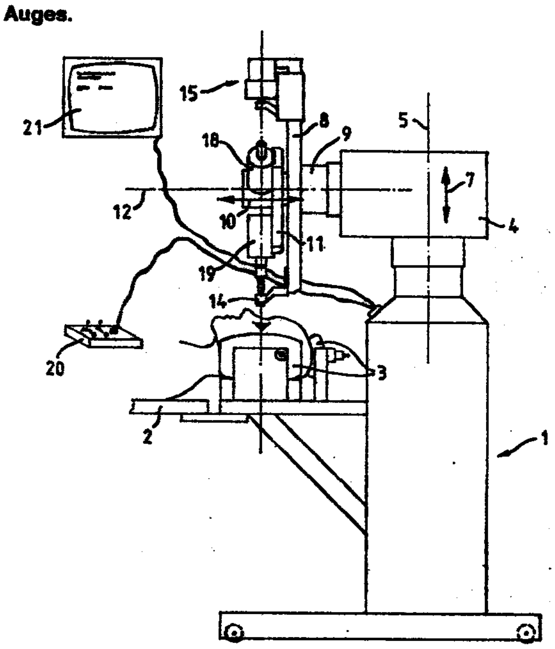

**EP 0 208 950 A2**

Anmelder: Dr. Patrik Gründler
Iddastraße 7
CH-9098 St. Gallen

amtl. Bez.: "Vorrichtung zur Transplantation der Cornea des menschlichen Auges"

Die Erfindung betrifft eine Vorrichtung zur Transplantation der Cornea des menschlichen Auges.

Aus der Patentliteratur bekannte Vorrichtungen sind jeweils nur für die Durchführung bestimmter Schritte oder für die Lösung von Teilaufgaben im Rahmen des gesamten ophtalmo-chirurgischen Prozesses bestimmt oder geeignet. Insbesondere wird hingewiesen auf:

Europäische Patentanmeldung, Veröffentlichungs-Nr. 0 151 869, Anmeldetag: 16. November 1984. Beschrieben wird ein festsaugbarer Bulbushaltering, der zur Fixierung des Auges bei der gesteuerten Fotodekompositon von bestimmten Bereichen der Cornea dient. Dieser Haltering ist jedoch nicht speziell auf die Erfordernisse der perforierenden Keratoplastik abgestimmt.

US-Patent 3 074 407, Anmeldetag: 17. September 1956. Beschrieben wird eine Trepanationsvorrichtung mit handbetätigtem Rohrmesser, das einen Ringspalt zwischen einem inneren und einem äußeren Saugfuß durchsetzt und bezüglich dieser ineinandergesteckten Saugfüße axial geführt ist. Der Nachteil dieser Vorrichtung besteht darin, daß sie nicht exakt genug zur optischen Achse des Auges zentriert werden kann. Beim Betrieb dieser Trepanationsvorrichtung besteht die Gefahr der Verletzung von Binnenstrukturen des Auges, weil die Vorrichtung gegen den Kopf des Patienten nicht eindeutig festgelegt ist.

Der heutige Stand der chirurgischen Technik bei der Hornhauttransplantation läßt sich etwa wie folgt darstellen:

Mit Hilfe eines eventuell motorisierten Rohrmessers wird von Hand ein kreisförmiger Einschnitt an der Cornea angebracht und sodann von Hand mittels Schere und Pinzette die zuvor umgrenzte Scheibe ganz ausgeschnitten und weggenommen. Die zu transplantierende Corneascheibe wird mittels eines Stanzwerkzeugs oder ebenfalls freihändig mit der Schere ausgeschnitten. Das Transplantat schließlich wird mit Nadel und Faden ebenfalls freihändig mittels feiner Nähte im Empfängerauge befestigt.

Obwohl bei allen diesen Vorgängen unter dem Operationsmikroskop gearbeitet wird, verlangt diese Vorgehensweise eine außergewöhnliche Handfertigkeit, welche nur sehr wenige und erfahrene Spezialärzte besitzen. Die Dauer der Operation beträgt im günstigsten Fall immer noch mehrere Stunden.

Für die Manipulation des sehr empfindlichen Transplantats, das einen Durchmesser von etwa 8 mm hat, wurden bisher keine besonderen Vorrichtungen verwendet. Das Transplantat wurde vielmehr mit einer Pinzette gefaßt. Die dadurch verursachte Traumatisierung des Transplantats verzögert die Heilung und verschlechtert ebenfalls das Operationsresultat.

Die Erfindung hat das Ziel, eine Vorrichtung vorzuschlagen, die als integriertes System durch sinnvolles und aufeinander abgestimmtes Zusammenwirken ihrer einzelnen Teile die Hornhauttransplantation am menschlichen Auge vereinfacht. Unabhängig von der Handfertigkeit des Operateurs soll ein reproduzierbares perfektes Resultat erreicht werden und gleichzeitig die Operationszeit beträchtlich reduziert werden, beipsielsweise auf 10 Minuten.

Die angestrebte Vorrichtung soll eine bessere Übereinstimmung der Mittelachse der Schnittkreisebene mit der optischen Achse des Empfängerauges ermöglichen und die Vorbereitung der Empfänger-Cornea zur Transplantation zeitlich beschleunigen.

Es ist ein weiteres Ziel der Erfindung, eine mit apparativen Hilfsmitteln entnommene und in Position gehaltene Corneascheibe schnell, zuverlässig und ohne Verformungen in der Empfänger-Cornea derart zu befestigen, daß sie rasch anwachsen und verheilen kann, wobei das Operationsergebnis nicht von der manuellen Geschicklichkeit des Arztes abhängig sein soll.

Ein weiteres Ziel der Erfindung besteht darin, die Operation mit solchen apparativen Hilfsmitteln durchzuführen, daß das Transplantat nicht von Hand, beispielsweise mittels einer Pinzette, erfaßt und bewegt werden muß.

Schließlich zielt die Erfindung darauf ab, die Vorrichtung so auszubilden, daß die mit dem Auge und dem Transplantat in Berührung kommenden Teile leicht abgenommen und sterilisiert werden können und von den nicht sterilen Teilen einen ausreichenden Abstand haben.

Ausgehend von der einleitend beschriebenen Vorrichtung werden diese Ziele durch die im Anspruch 1 gekennzeichneten Merkmale erreicht. Diese Vorrichtung mit ihrem dreidimensional beweglichen Instrumententräger bewegt sämtliche Instrumente unter der Kontrolle des Arztes und von ihm feinfühlig gesteuert mittels elektronisch gesteuerter Antriebe. So sind diese Bewegungen viel genauer als ein Mensch sie von Hand ausführen könnte. Ist eines der Instrumente einmal auf die optische Achse des Empfängerauges eingestellt, so kann infolge der Speichermöglichkeit der Stellungskoordinaten auch ein anderes Instrument auf Knopfdruck in diese Stellung gefahren werden. Ein wesentlicher Vorteil besteht auch darin, daß die Vorrichtung über eine Steuereinheit (Rechner) programmiert werden kann, so daß die einzelnen bei der Operation erforderlichen Tätigkeiten unter der Kontrolle des Operateurs automatisch ablaufen, wodurch sich eine Verkürzung der Operationszeit auf etwa 10 Min. erreichen läßt.

Aus konstruktiven Gesichtspunkten ist es besonders vorteilhaft, den Träger als um eine horizontale Achse gelagerten Drehteller auszubilden, an dem die Instrumente sternförmig mit radialen Mittelachsen angeordnet sind. Dieser Drehteller ist an einem Hilfsträger gelagert, der zusammen mit dem Drehteller in Richtung der Drehtellerachse horizontal verschiebbar, um eine vertikale Achse drehbar und in Richtung dieser vertikalen Achse verschiebbar ist. Der Hilfsträger führt somit die dreidimensionale Bewegung aus (Höhe, Radius und Winkel), während die Drehbewegung des Drehtellers lediglich zum Wechsel der Instrumente zwischen dem Empfängerauge und den sonstigen apparativen Komponenten dient, die am Hilfsträger befestigt sind.

An dem Hilfsträger ist der Bulbushaltering in vertikaler Richtung verschiebbar und genau einstellbar angebracht, derart, daß das Achseinstellinstrument, der Trepan und der Transplantator koaxial zum Bulbushaltering eingestellt werden können. Der Trepan und der Transplantator sind feinfühlig radial zur Drehtellerachse verstellbar und hinsichtlich ihres Außendurchmessers am radial äußeren Ende so bemessen, daß sie passend in die Öffnung des Bulbushalterings eingefügt werden können.

Um den Operationsvorgang besser beobachten zu können, kann der Bulsbushaltering aus einem durchsichtigen Werkstoff gefertigt sein. Es können aber auch Lichtleiter oder flexible optische Faserbündel in den Bulbushaltering eingesetzt sein, die ihn von außen nach innen durchdringen. Auf diese Weise läßt sich der Operationsvorgang von der Vorderkammerseite her unter direkter Beleuchtung beobachten.

An dem Hilfsträger ist ferner vorzugsweise ein Manipulator angebracht, welcher zum Halten der Spendercornea und des Transplantats, zum Übertragen desselben auf den Transplantator und zum Schutz des Transplantats zwischen den einzelnen Operationsschritten dient. Der Manipulator kann auch eine Vorrichtung aufweisen, die es gestattet, das Transplantat im Randbereich mit einem biokompatiblen Kleber (vorzugsweise Fibrinogen) zu benetzen. Schließlich ist an dem Hilfsträger ein Klammerpatronen-Wechsler angebracht, welcher mit der noch zu beschreibenden Klammervorrichtung des Transplantators zusammenwirkt und diesen selbsttätig mit neuen Klammern zum Befestigen des Transplantats versorgt.

Das Achseinstellinstrument ist prinzipiell ein monokulares Mikroskop mit Beleuchtung zur Betrachtung des Augenhintergrundes und weist eine im Okular im Sehfeldzentrum erscheinende Markierung auf. Hinter und vor dem Linsensystem sind halbdurchlässige Spiegel angeordnet, mittels derer ein Teil der Strahlung abgezweigt und dem Hauptstrahlengang wieder zugefügt wird, wobei dieser zweite Strahlengang durch ein zweites Linsensystem verläuft, das ein scharfes Bild von der Corneaoberfläche erzeugt. Vor der Operation wird im Zentrum der zu entfernenden Cornea eine kleine Markierung angebracht. Daraufhin läßt sich die angestrebte Übereinstimmung der Sehachse und der optischen Achse des Achseinstellinstruments dadurch herbeiführen, daß man den Instrumententräger, d. h. den Hilfsträger und den Drehteller, in der Horizontalen derart verfährt und das Patientenauge derart ausrichtet, daß die Sehfeldmarkierung, die Corneamarkierung und die am Augenhintergrund sichtbare Makula Lutea miteinander zur Deckung kommen. Zum Zwecke einer unbehinderten und raschen Durchführung der Einstellarbeiten kann am Achseinstellinstrument eine Viedeokamera angeordnet sein, welche den Einstellvorgang auf einem Monitor sichtbar macht.

Ein geeigneter Trepan besteht im wesentlichen aus einem Halter und einem Schaft, an dessen Ende ein Saugfuß in Gestalt eines gasdurchlässig ausgebildeten Formkörpers befestigt ist, welcher eine der äußeren Oberflächenform der menschlichen Cornea angenäherte Anlagefläche aufweist. Der Schaft ist umgeben von einem Rohrmesser, das vorzugsweise elektrisch angetrieben wird und bezüglich des Schaftes verschiebbar und genau einstellbar ist, insbesondere hinsichtlich der Endbegrenzung der Schneidtiefe. Ein derart einfacher Trepan ist deshalb vorzuziehen, weil er eine gute Sichtkontrolle gestattet. Die Trepanation erfolgt in der Weise, daß zunächst der Saugfuß bis zum genauen Aufliegen auf der Corneaoberfläche abgesenkt wird, was von der Seite her beobachtet werden kann, und sodann durch selbsttätigen Stellungsvergleich der Bulbushaltering auf die entsprechende Stellung abgesenkt wird, welcher von da an den Bulbus ohne Druck und Verformung durch Ansaugen des corneal-skleralen Bereichs während der ganzen Operation festhält. Sodann kann die Trepanation gefahrlos selbsttätig durchgeführt werden.

Zur Erhöhung der Sicherheit gegen zu starkes Vakuum oder sonstige Zwischenfälle bei der Trepanation wird vorgeschlagen, daß der Saugfuß oder seine Vakuum-Anschlußleitung einen feuchtigkeitsempfindlichen Sensor aufweist, der das angelegte Vakuum verringert und ein Signal auslöst, falls Kammerflüssigkeit angesaugt wird.

Ein besonders geeignetes Rohrmesser, welches praktisch keine Kraft auf die Cornea ausübt und ein einwandfreies Schnittergebnis liefert, hat an seiner Schnittkante Zacken mit angeschliffenen Schneidfassetten, die bei der Drehbewegung vorauslaufen.

Der Transplantator dient erfindungsgemäß dazu, das Transplantat in die Empfängercornea passend einzusetzen und vorübergehend zu befestigen, bis der Heilungsprozeß abgeschlossen ist. Der Transplantator besteht im wesentlichen aus einem Halter, der die gleichen Bewegungsmöglichkeiten bezüglich des Drehtellers hat wie der Trepan, einem Schaft und einem am Ende des Schaftes befindlichen Saugfuß. Der Saugfuß hat im Gegensatz zu demjenigen des Trepans zwei Zonen, nämlich eine Innenzone und eine diese umgebende äußere Ringzone, die an getrennt regulierbare Vakuumquellen angeschlossen sind. Die Innenzone des Saugfußes faßt das Transplantat und die Ringzone den mit dem Transplantat zu verbindenden Trepanationsrand. Hierzu sei bemerkt, daß der Außendurchmesser dieser ringförmigen Saugzone sich innerhalb des Bulbushalterings befindet, da der Transplantator in den Bulbushaltering eingeführt werden kann. Der Saugfuß enthält ferner wenigstens einen in seine Anlagefläche, d. h. auf die Cornea, mündenden Klammerkanal, in welchem sich ein Stempel bewegt, der eine Klammer ausstößt und dabei in die Cornea eindrückt. Vorzugsweise sind mehrere Klammerkanäle vorgesehen, deren Mündungsöffnungen im Bereich zwischen der Ringzone und der Innenzone kreisförmig angordnet sind. Die Klammerkanäle verlaufen vorzugsweise in Schaftlängsrichtung, so daß die überstehenden Stempel mittels eines einzigen Schlagorgans gemeinsam betätigbar sind.

Da die Kraft zum Eindrücken der Klammern kleiner sein muß als die Saugkraft des Saugfußes, ist die Zahl der gleichzeitig einzudrückenden Klammern begrenzt, aber auch aus konstruktiven Gründen. Es wird deshalb vorgeschlagen, in mehreren Vorgängen unter sukzessiver Verdrehung des Saugfußes um die Transplantatorachse mehrere Klammersätze einzubringen, so daß diese im fertigen Zustand eine regelmäßige kreisförmige Anordnung aufweisen. Zu diesem Zweck, aber auch zur Sicherstellung der Sterilisierung, wird vorgeschlagen, daß der Fußteil des Schaftes, der Saugfuß und die Stempel eine Klammerpatrone bilden, die mittels einer Schnellkupplung leicht vom Schaft abnehmbar und wahlweise in unterschiedlichen Winkelstellungen ansetzbar ist, wobei die Schlagkraft mittels einer längsbeweglichen Hülse auf die Stempel übertragen wird. Die Schnellkupplung kann beispielsweise ein Bajonettverschluß sein.

Der schon erwähnte, am Hilfsträger angebrachte Klammerpatronen-Wechsler besorgt das Abnehmen der leeren Klammerpatrone und das Ansetzen einer neuen Klammerpatrone selbsttätig. Er enthält einen Schacht, in welchen eine Magazinpackung mit mehreren sterilisierten Klammerpatronen eingefügt werden kann. In der Magazinpackung sind die Klammerpatronen in der gewünschten unterschiedlichen Winkelorientierung enthalten. Es ist ferner eine mechanische Wechselvorrichtung mit einem längsverschiebbaren und drehbaren Greifer vorgesehen, welcher die Klammerpatronen erfaßt und dreht, wobei die neuen Klammerpatronen dem Greifer selbsttätig zugefördert und die leeren Patronen ausgeworfen werden. Ist ein Klammersatz eingedrückt, so fährt der Transplantator nach Abschalten seiner Vakuumleitungen aus dem Bulbushaltering heraus und bewegt sich durch Drehen des Drehtellers in eine dem Klammerpatronen-Wechsler koaxial gegenüberliegende Stellung. Mit einer neuen Klammerpatrone ausgestattet bewegt er sich dann wieder zurück und setzt diese weiteren Klammern in das Transplantat bzw. den Randbereich der Empfängercornea ein.

Vorzugsweise werden U-förmige Klammern mit einem Verbindungssteg und zwei parallelen Schenkeln verwendet, die nach innen weisende Widerhaken an den Schenkeln aufweisen. Die Schenkel können auch zum Verbindungssteg schräg stehen. Als Werkstoff für die Klammern kommt ein geeigneter Kunststoff oder Metall in Betracht. Der Querschnitt kann flach, rund oder halbrund sein. Mit entsprechenden anderen Klammerapparaten lassen sich auch V-förmige Klammern verwenden, wobei nur an einem Schenkel ein Widerhaken angeordnet ist. Solche Klammern werden in einem sehr spitzen Winkel bezüglich der Corneaoberfläche in die Verbindungsstelle der Empfängercornea mit dem zu befestigenden Transplantat radial eingedrückt, so daß der eine Schenkel die Schnittflächen nahezu senkrecht durchsetzt. Dieser Schenkel kann auch kreisförmig gebogen sein, in welchem Falle diese Klammer in einer Drehbewegung bezüglich des Krümmungszentrums dieses Schenkels eingebracht werden muß. Der mit Widerhaken versehene zweite Schenkel, der außen auf der Corneaoberfläche aufliegt, verhindert mit dem Widerhaken das radiale Herausrutschen der Klammer. Dieser außen aufliegende Schenkel sollte der Corneawölbung entsprechend gebogen sein, um ein Fremdkörpergefühl des Patienten zu vermeiden.

Die Schlagkraft wird vorzugsweise elektromagnetisch oder pneumatisch erzeugt. Es kommt aber auch eine Feder als Schlagkraftantrieb in Betracht, welche elektromagnetisch gespannt und ausgelöst wird, oder eine Betätigung mit Hilfe kleiner Sprengkapseln. Um die Erschütterungen gering zu halten, wird vorgeschlagen, die Schlagkraft mittels eines im Zentrum des Schaftes geführten Stabes auf die Stempel zu übertragen und dadurch die bewegte Masse zu reduzieren. Andererseits kann ein Gegengewicht vorgesehen sein, das mit dem impulsgebenden Schlagstempel so gekoppelt ist, daß es sich gegenläufig zu diesem bewegt und die Impulse beider Massen sich beim gleichzeitigen Auftreffen auf ihre Anschläge neutralisieren.

Um schließlich jede Berühung des Transplantats durch ein handgeführtes Werkzeug zu vermeiden, ist der erwähnte Manipulator am Hilfsträger vorgesehen. Er besteht aus einem Transplantatgreifer, einem horizontal angeordneten Corneateller und einer Sicherheitsschale, welche um eine gemeinsame vertikale Achse drehbar gelagert sind, wobei die eine Konservierungsflüssigkeit, z. B. physiologische Kochsalzlösung, enthaltende Sicherheitsschale außerdem in Höhenrichtung verfahrbar ist, so daß der Transplantatgreifer teilweise in die Flüssigkeit eintauchen kann. Der Corneateller hat eine dem Außendurchmesser des Trepans entsprechende zentrale Öffnung und eine diese umgebende ringförmige Auflagefläche, welche als gasdurchlässige Saugzone ausgebildet und an ein Vakuum angeschlossen ist. Der Corneateller dient als Auflage für die Spendercornea mit anhaftenden Gewebeteilen, wobei sich der auszuschneidende zentrale Bereich mit seiner Außenseite nach unten gekehrt über der Öffnung befindet. Das Transplantat wird mit dem Trepan von unten kommend ausgeschnitten. Danach fährt der Trepan mit dem auf seinem Saugfuß liegenden Transplantat nach oben zum Transplantatgreifer, der mehrere sternförmig angeordnete spitze, korkenzieherförmige Tragelemente aufweist, die sich radial bewegen und sich in die Schnittflächen des Transplantats einbohren und dieses somit halten, um es anschließend auf den Transplantator aufzulegen.

Ein Ausführungsbeispiel der Erfindung wird nachfolgend anhand der Zeichnungen erläutert und sodann die Wirkungsweise der einzelnen Komponenten im Zusammenhang durch Beschreibung des Ablaufs der Operation dargestellt.

Im einzelnen zeigt

Fig. 1 die Seitenansicht einer integrierten Vorrichtung zur Hornhauttransplantation in schematischer Darstellung,

Fig. 2 die Draufsicht der Vorrichtung nach Fig. 1,

Fig. 3 die Ansicht des Drehtellers der Vorrichtung nach Fig. 1 in Achsrichtung,

Fig. 4 einen schematischen Axialschnitt eines Achseinstellinstruments,

Fig. 5 einen Axialschnitt eines motorisch angetriebenen Trepans,

Fig. 6 einen Axialschnitt eines Bulbushalteringes,

Fig. 7 einen Axialschnitt des Bulbushalteringes mit eingefahrenem Trepan in größerem Maßstab,

Fig. 8 eine Ansicht der Anlagefläche des Saugfußes des Trepans in zwei Ausführungsvarianten,

Fig. 9 eine Seitenansicht eines Ausschnitts aus dem unteren Teil des Rohrmessers in weiter vergrößertem Maßstab,

Fig. 10 einen Axialschnitt eines Transplantators mit Saugfuß und Klammervorrichtung,

Fig. 11 einen Axialschnitt des unteren Teils des Transplantators in größerem Maßstab,

Fig. 12 eine Ansicht der Auflagefläche einer anderen Ausführungsform des Transplantator-Saugfußes gemäß Fig. 11,

Fig. 13 eine Ansicht verschiedener Klammern in größerem Maßstab,

Fig. 14 eine schematische Darstellung eines Klammerpatronen-Wechslers,

Fig. 15 einen Axialschnitt eines Manipulators und

Fig. 16 einen vergrößerten Ausschnitt XVI aus Fig. 15.

Die Vorrichtung nach den Figuren 1 bis 3 weist ein Grundgestell (1) auf, das im wesentlichen aus einer verfahrbaren Basis, einem Turm und einer daran befestigten Tischkonsole besteht, an welche eine Patientenliege (2) angekoppelt ist. Auf der Tischkonsole befindet sich eine Auflage mit Druckpolstern (3) zur Fixierung des Kopfes des Patienten. Diese Fixiervorrichtung kann im übrigen auch den bei stereotaktischen Hirnoperationen verwendeten Vorrichtungen entsprechen und Haltestifte oder dgl. aufweisen.

In dem Turm ist eine Dreh- und Hubvorrichtung eingebaut, welche einen Turmkopf (4) bezüglich der vertikalen Turmachse (5) in Pfeilrichtung (6) (Fig. 2) dreht und in Pfeilrichtung (7) hebt und senkt. In dem Turmkopf (4) ist ein Hilfsträger (8) mittels eines Schieberohres (9) in Richtung des Pfeils (10) horizontal verschiebbar. Diese Translationsbewegung macht auch ein Drehteller (11) mit, der in dem Hilfsträger (8) und dem Schieberohr (9) um eine horizontale Achse (12) in Pfeilrichtung (13) drehbar gelagert ist. Der Hilfsträger (8) kann sich also nicht drehen und der Drehteller (11) kann sich bezüglich des Hilfsträgers (8) nicht verschieben.

An dem T-förmigen Hilfsträger (8) ist unten ein Bulbushaltering (14) getrennt höhenverschiebbar angebracht. Oben trägt der Hilfsträger (8) einen Manipulator (15) und an dem seitlichen Arm des Hilfsträgers ist ein Klammerpatronen-Wechsler (16) befestigt. Der Drehteller (11) trägt in sternförmiger Anordnung drei Instrumente, nämlich ein Achseinstellinstrument (17), einen Trepan (18) und einen Transplantator (19). Die beiden zuletzt genannten Instrumente sind bezüglich der Achse (12) des Drehtellers (11) radial verstellbar.

Alle vorgenannten rotatorischen und translatorischen Bewegungen sind von hochgenauen elektronischen Antrieben gesteuert. Vorzugsweise sind die Stellungen digital ablesbar und speicherbar, so daß eine einmal festgelegte Stellung einer bestimmten Komponente nach zwischenzeitlicher Bewegung wieder aufgefunden werden kann. Insbesondere ist es möglich, den Drehteller (11) in bevorzugte Winkelstellungen einzustellen, so daß die einzelnen Instrumente wahlweise eine 6-Uhr-, 9-Uhr- oder 12-Uhr-Stellung einnehmen, wobei diese Instrumente (gemäß Fig. 3) nach unten, nach links oder nach oben weisen. Durch entsprechende Steuerprogramme kann auch erreicht werden, daß die Instrumente im Anschluß an eine befohlene Drehbewegung des Drehtellers (11) eine Translationsbewegung ausführen, wobei die gewünschte Endstellung vorher mittels eines anderen Instruments ermittelt worden ist. Die Bezugspunkte für die Translationsbewegungen der einzelnen Instrumente sowie auch des Bulbushalterings (14) sind ebenfalls einstellbar und gegeneinander abgleichbar.

Dem Operateur steht eine Steuerkonsole (20) und ein Video-Monitor (21) zur Verfügung, die an der Vorrichtung angeschlossen sind. Die Steuerkonsole enthält im wesentlichen einen Steuerknüppel für die gemeinsame Horizontalbewegung des Hilfsträgers (8) und des Drehtellers (11) in den Richtungen (6 und 10), einen Schalter für die Hubbewegung des Turmkopfes (4) in Richtung (7), vorzugsweise für zwei Geschwindigkeiten, Betätigungsorgane für die einzelnen Instrumente und den Bulbushaltering sowie für die verschiedenen Programme. Schließlich enthält die Steuerkonsole (20) noch einen Not-Aus-Knopf, dessen Betätigung zur Folge hat, daß sämtliche Vakuumleitungen belüftet werden, Motorbewegungen stoppen oder sämtliche Komponenten nach oben fahren.

Das Achseinstellinstrument (17) nach Fig. 4 hat einen längeren Tubus mit einem Linsensystem (28) sowie einen parallelen kürzeren Tubus mit einem zweiten Linsensystem (29). In dem geradlinigen Hauptstrahlengang (30) ist ein halbdurchlässiger Spiegel (31) unter 45° angeordnet, mittels dessen über einen weiteren Spiegel (32) das Licht einer Beleuchtungsquelle (33) eingeführt wird. Mit diesem insoweit bekannten Instrument läßt sich der Augenhintergrund beobachten, wobei die Vergrößerung so gewählt sein sollte, daß es möglich ist, in einem ausreichend großen Sehfeld die Umrisse der Makula (34) klar zu erkennen.

Im Hauptstrahlengang (30) sind außerdem zwei weitere halbdurchlässige Spiegel (35 und 36), die rechtwinklig zueinander stehen, unter 45° eingebaut. Mit ihrer Hilfe wird ein Parallelstrahlengang (37) durch das Linsensystem (29) geschaffen. Mit Hilfe dieses selbstverständlich getrennt einstellbaren Linsensystems läßt sich die Oberfläche der Cornea (38) und somit auch eine in deren Zentrum zuvor angebrachte kleine Markierung (39) scharf beobachten. Schließlich ist im Hauptstrahlengang ein Fadenkreuz (40) oder eine ähnliche Markierung angeordnet. Man kann somit durch achsparalleles Verstellen des Achseinstellinstruments und durch Korrektur der Stellung des Patientenauges, beispielsweise durch Manipulation von zwei am Bulbus befestigten Haltefäden (41), das Fadenkreuz (40), die Markierung (39) und die Makula (34) im Bild zur Deckung bringen.

Um dem Arzt die erforderliche Bewegungsfreiheit bei diesem Einstellvorgang zu geben, ist dem Okular vorgeschaltet bzw. an dessen Stelle eine Videokamera (42) vorgesehen. Mit ihrer Hilfe kann der Einstellvorgang am Monitor (21) beobachtet werden. Es kann zweckmäßig sein, eine sog. Rohrkamera zu verwenden und diese aus Platzgründen nicht in geradliniger Verlängerung des Instruments sondern in dem horizontalen Schieberohr (9) anzuordnen, wobei der Strahlengang einmal umgelenkt werden muß.

Der in den Figuren 5 und 7 gezeigte Trepan ist an dem Drehteller (11), der nur bruchstückhaft dargestellt ist, in Richtung des Pfeils (50) genau parallel zur Mittelachse (51) eines Rohrmessers (52) verstellbar gelagert. Das Rohrmesser hat am oberen Ende eine ringförmige Verdickung (53) von trapezförmigem Querschnitt, und ist mittels einer Rändelmutter (54) mit der hohlen Welle (55) des Rotors (56) eines Elektromotors verbunden. Der Rotor ist mittels zweier Lager (57) in einem Stator (58) gelagert, der als Schieber ausgebildet ist und sich in Richtung des Pfeiles (59) bezüglich eines Halters (60) ebenfalls genau parallel zur Rohrmesserachse (51) bewegen kann. Der Halter (60) ist seinerseits - wie erwähnt - am Drehteller (11) verschiebbar gelagert. Die Bewegung des Stators (58) gegenüber dem Halter (60) bewirkt den vertikalen Vorschub des Rohrmessers (52) bei der Schneidbewegung und beträgt nur wenige Millimeter.

Der Halter (60) ist fest verbunden mit einem Schaft (61), der sich koaxial durch den Rotor (56) und das Rohrmesser (52) hindurch erstreckt und an seinem unteren Ende einen zylindrischen Saugfuß (62) trägt. Der untere Abschnitt (63) des Schaftes mit dem Saugfuß (62) ist mittels einer Schraubkupplung an den Schaft (61) angeschlossen. Von dem Saugfuß (62) erstreckt sich eine zentrale Saugleitung (64) durch den Schaft (61) hindurch. Sie ist oben über ein Ventil (65) an eine Vakuumquelle angeschlossen. Der Außendurchmesser des Saugfußes (62) und der Innendurchmesser des Rohrmessers (52) sind so aufeinander abgestimmt, daß sich das Rohrmesser drehen und in Achsrichtung verschieben kann, wobei jedoch das Lagerspiel so gering wie möglich gehalten ist.

Der in Fig. 6 dargestellte Bulbushaltering (14) ist über einen Arm (66) mit einem Schlitten (67) verbunden, welcher bezüglich des während der Operation stillstehenden Hilfsträgers (8) in Richtung des Pfeiles (68) beweglich ist, und zwar genau parallel zur Achse (69) der Bohrung des Bulbushalterings. Wie Fig. 7 zeigt, paßt das Rohrmesser (52) mit dem Außendurchmesser seines mittleren Abschnitts genau in diese Bohrung. Die untere sog. Anlagefläche (70) des nach unten konisch zulaufenden Bulbushalterings (14) hat eine nach innen ansteigende flach konische Form. Sie weist einen ringförmigen schmalen Saugschlitz (71) auf, der über nach oben verlaufende Bohrungen (72) und einen ringförmigen Sammelkanal (73) einer Saugleitung (74) verbunden ist, die über ein besonderes Ventil (75) zur Vakuumquelle führt.

In Fig. 7 ist der Bulbushaltering (14) größer dargestellt. Die linke Seite dieser Figur zeigt im Schnitt faseroptische Beobachtungsmittel, die an wenigstens einer Stelle des Umfangs in den Ring eingesetzt sind. An dieser Stelle kann der Saugschlitz (71) unterbrochen sein. Am inneren unteren Rand des Bulbushalterings (14) befindet sich ein kleines optisches Prisma (147). Zu der nach links oben weisenden Fläche dieses Prismas führt ein Lichtleiter (148) und ein optisches Fasterbündel (149), welch letzteres an eine Videokamera angeschlossen ist. Das Prisma lenkt die aus dem Lichtleiter austretenden Strahlen zur Ringmitte, so daß sie durch den cornealen Randbereich hindurchtreten und das Operationsfeld beleuchten. Durch das Faserbündel (149) kann am angeschlossenen Monitor der Operationsvorgang beobachtet werden.

Die Figuren 7 bis 9 zeigen auch weitere Einzelheiten der wirksamen Teile des Trepans. Der Saugfuß (62), der in eine dünnwandige Ausnehmung des Schaftabschnitts (63) eingesetzt ist, ist im Beispiel ein poröser Formkörper. Die linke Hälfte der Fig. 8 stellt ebenfalls diese Ausführungsform dar. Der Saugfuß könnte aber auch ein massiver, von einer Vielzahl von Saugbohrungen (76) durchzogener Körper sein, die in seiner Anlagefläche (77) münden. Dieses Beispiel ist in der rechten Hälfte der Fig. 8 dargestellt. Die Anlagefläche (77) ist konkav und hinsichtlich ihrer Krümmung möglichst genau der Außenwölbung der Cornea (78) angepaßt. In unmittelbarer Nähe des Saugfußes (62) ist in der Saugleitung (64) ein Feuchtigkeitssensor (79) eingebaut, welcher die Aufgabe hat, nach der Trepanation eventuell angesaugtes Kammerwasser sofort festzustellen und zu melden und über das Ventil (65) eventuell das Vakuum selbsttätig zu reduzieren oder abzuschalten.

Der längere Mittelabschnitt des Rohrmessers (52) liegt an der zylindrischen Bohrung des Bulbushalterings (14) an, so daß sich diese beiden Teile gegenseitig führen. Der untere Abschnitt (80) des Rohrmessers ist außerordentlich dünnwandig ausgebildet und wird nur von innen durch den mit seiner Außenfläche anliegenden Schaftabschnitt (63) geführt. In der linken Hälfte der Fig. 7 befindet sich das Rohrmesser in seiner zurückgezogenen Bereitschaftsstellung. In der rechten Hälfte dagegen ist das Rohrmesser bis zur völligen Durchtrennung der Cornea ausgefahren. Das ausgeschnittene Corneascheibchen hängt am Saugfuß und wird beim Hochfahren des Trepans mitgenommen.

Fig. 9 zeigt in Vergrößerung den Schneidrand des Rohrmesserabschnitts (80). An den Flanken der sägezahnförmigen Zacken (81) sind Schneidfacetten (82) angeschliffen. Die Drehrichtung des Rohrmessers ist so gewählt, daß sich der dargestellte Schneidrand in Richtung des Pfeiles (83) bewegt.

Die Figuren 10 bis 13 zeigen den Transplantator (19) mit der an ihm angebauten Klammervorrichtung. Die Vorrichtung insgesamt gemäß Fig. 10 besteht aus einem Halter (90) der am Drehteller (11) in Richtung des Pfeiles (91) verschiebbar angebracht ist. Die Verschieberichtung verläuft parallel zur Achsrichtung eines Schaftes (92), der mit dem Halter (90) fest verbunden ist. Der Schaft durchsetzt eine Hülse (93), die an den Halter (90) angeformt ist und nach unten ragt. Zwischen der Hülse (93) und dem Schaft (92) befindet sich eine in Längsrichtung lose bewegliche Schlaghülse (94).

Auf die Hülse (93) ist von unten eine Klammerpatrone (95) mit Hilfe eines Bajonettverschlusses (96) aufgesteckt. Der Bajonettverschluß hat wenigstens sechs über den Umfang verteilte Stifte, so daß die Klammerpatrone wahlweise in drei verschiedenen Winkelstellungen angesetzt werden kann. Ein zylindrischer Ansatz (97) am unteren Ende der Klammerpatrone paßt ebenfalls in die Bohrung des Bulbushalterings (14). Im Hinblick auf den Zweck der Klammerpatrone, sowohl das in die Empfängercornea einzusetzende scheibchenförmige Transplantat als auch den Rand der ausgeschnittenen Empfängercornea in der richtigen Position festzuhalten und durch Klammern miteinander zu verbinden, hat der Ansatz (97) zwei getrennte Saugvorrichtungen, nämlich einen zentralen Saugfuß (98) und einen ihn umgebenden Saugring (99). Beide sind an getrennte Saugleitungen (100) und (101) angeschlossen, welche durch den Schaft (92) verlaufen und über Ventile (101 und 103) an die Vakuumquelle angeschlossen sind.

Die Saugleitungen verlaufen auch durch die Klammerpatrone und zwar durch einen zentralen, nach oben weisenden Kern, der in eine stirnseitige Ausnehmung des Schaftes (92) paßt und dadurch die Saugleitungsabschnitte dicht verbindet.

In dem Ringbereich zwischen den beiden Sauganordnungen verlaufen in dem Ansatz (97) in Längsrichtung acht Klammerkanäle, welche von passenden längsbeweglichen Stempeln (104) ausgefült sind. Die Stempel haben breitere Mittelabschnitte, die sich durch entsprechend vergrößerte Kanäle im mittleren Bereich der Klammerpatrone (95) erstrecken und oben einen abermals verbreiterten Endabschnitt (105) aufweisen. Kleine Druckfedern (106) halten die Stempel in einer oberen Ruhestellung. Die Kraft zum Eintreiben der Klammern kommt im Beispiel von einem Antriebsring (107), welcher auf dem Schaft (92) verschiebbar gelagert ist und infolge seines nicht dargestellten elektromagnetischen Antriebs oder dgl. in Richtung des Pfeiles (108) nach unten fährt und die Schlaghülse (94) mitnimmt. Das untere Ende der letzteren legt sich gegen die Endabschnitte (105) der Stempel und bewegt diese somit ebenfalls gemeinsam nach unten.

Die Fig. 11 zeigt weitere Einzelheiten des Transplantators. Der innere Saugfuß (98) ist ein poröser Körper. Am Beginn der Saugleitung (100) befindet sich ebenfalls ein Feuchtigkeitssensor (109). Der Saugring (99) besteht aus einer Vielzahl von im Außenbereich des Ansatzes (97) in Längsrichtung geführten Saugkanälen (110), die über eine Ringleitung der Saugleitung (101) zugeführt sind. Fig. 12 zeigt die Ansicht der gesamten Sauganordnung von unten, wobei als Alternative und im Gegensatz zu Fig. 11 auch im zentralen Bereich querschnittlich runde Saugkanäle verwendet sind. Fig. 12 zeigt auch den länglichen Querschnitt und die sternförmige Anordnung der acht Klammerkanäle (111). Wesentlich ist, daß auch zwischen den Klammerkanalöffnungen Saugkanäle angeordnet sind, so daß in diesen Bereichen das Transplantat (112) und die dieses umgebende Empfängercornea bis an die Ränder heran sauber gehalten sind. Zur einfacheren Darstellung sind die Öffnungen der Saugkanäle (110) des äußeren Saugrings schwarz ausgefüllt und die übrigen Saugöffnungen als Kreise dargestellt. Im innersten Bereich sind die Saugöffnungen etwas größer. Die Öffnungen der die Klammerkanäle (111) umgebenden Sauganordnungen sind auch mit schwammartig gasdurchlässigen Körpern zu verwirklichen.

Eine der im Beispiel verwendeten Klammern (113) ist in vielfach vergrößertem Maßstab in Fig. 13 dargestellt. Sie besteht aus zwei parallelen Schenkeln und einem schrägen Steg. An den Innenseite der Schenkel sind Widerhaken angebracht. Die Klammer ist aus einem geeigneten Metall gefertigt und hat einen halbrunden Querschnitt.

Fig. 13 zeigt ferner eine V-förmige Klammer (113a). Der eine, etwas kürzere Schenkel ist geradlinig und der andere, etwas längere entsprechend der Oberflächenwölbung der Cornea leicht gekrümmt. Am Ende dieses letzteren Schenkels befindet sich eine nach innen weisende Spitze (Haken). Diese Klammer (113a) wird in der Weise eingesetzt, daß der geradlinige kürzere Schenkel radial durch die seitliche Schnittfläche dringt und der leicht gekrümmte Schenkel auf der Corneaoberfläche aufliegt, wobei die Spitze in die Oberfläche eindringt. Eine weitere Klammer (113b) unterscheidet sich von der zuvor beschriebenen dadurch, daß der erste Schenkel, der keinen Widerhaken aufweist, kreisförmig gekrümmt ist. Er muß dementsprechend bezüglich seines Krümmungszentrums auf einer Kreisbahn eingebracht werden.

Im linken Teil der Fig. 11 ist der Stempel (104) in Ruhestellung dargestellt. Sein unteres Ende ist bezüglich der Öffnung des Klammerkanals etwas zurückgezogen, so daß eine Klammer (113) mit dem Steg an der ebenfalls abgeschrägten Stirnfläche des Stempels (104) anliegend im Klammerkanal (111) Platz findet. So sind zu Beginn des Klammervorgangs bzw. bei den in Vorrat gehaltenen Klammerpatronen alle acht Klammerkanäle mit je einer Klammer geladen. Auf ein Signal stößt der Antriebsring (107) alle Stempel gemeinsam nach unten, so daß diese ihre Klammern (113) in das Gewebe eindrücken, was in der rechten Hälfte der Fig. 11 gezeigt ist. Die beim Übergang zur ersten Verbreiterung der Stempel gebildete Anschlagfläche stößt dabei gegen die obere Stirnfläche (114) des Ansatzes (97), wodurch die Stempelbewegung genau begrenzt ist. Die Länge der Klammerschenkel ist vorzugsweise geringer als die Dicke des Transplantats.

Sofern acht Klammern zur Befestigung des Transplantats nicht ausreichen, können in weiteren getrennten Vorgängen jeweils weitere acht Klammern winkelversetzt angebracht werden, so daß sich die Zahl der insgesamt gesetzten Klammern beliebig steigern läßt. Dieses Vorgehen ist auch dann von Bedeutung, wenn aus konstruktiven Erwägungen die einzelnen Klammerpatronen weniger Klammerkanäle haben, beispielsweise nur drei. Es müssen nur so viele sein, daß nach dem ersten Klammerungsvorgang das Transplantat vorübergehend und unter den Bedingungen der Operation seine Lage nicht verändert. Auch das Verhältnis Saugkraft zu Stempelkraft mag die Zahl der gleichzeitig einzubringenden Klammern nach oben begrenzen.

In Fig. 14 ist ein Klammerpatronen-Wechsler (16) gezeigt, der am Hilfsträger (8) befestigt ist und in einem Schacht (120) eine sterile Magazinpackung (121) aufnehmen kann.

Diese enthält drei Klammerpatronen (95) in um 15° unterschiedlichen Winkelstellungen, was durch Führungsstreifen (122) im Zusammenwirken mit einer entsprechenden Außenform der Klammerpatronen sichergestellt wird. In der Vorrichtung ist ferner ein Drehfutter (123) vorgesehen, das bezüglich seiner im Beispiel horizontalen Achse (124) gedreht und verschoben werden kann. Der Schacht (120) hat in Richtung der Achse (124) eine Öffnung (125).

Der Wechselvorgang verläuft wie folgt: Der Transplantator (19) wird durch Schwenken des Drehtellers (11) in die 9-Uhr-Stellung gebracht. Die Anordnung ist so getroffen, daß hierbei die Transplantatorachse mit der Achse (124) fluchtet. Durch Verschiebung des Transplantators in Richtung des Pfeiles (91) auf dem Drehteller wird die Klammerpatrone durch die Schachtöffnung (125) in den Wechsler eingeführt. Nun kommt von links das Drehfutter (123), faßt die Klammerpatrone und dreht den Bajonettverschluß auf. Jetzt wird der Transplanator etwas zurückgefahren und die leere Klammerpatrone vom Drehfutter seitlich ausgeworfen. Die oberste der Ersatzpatronen rückt nun unter Beibehaltung ihrer Winkelstellung nach oben an die Stelle der ausgeworfenen Patrone. Der Transplantator fährt wieder ein und das Drehfutter setzt die neue Patrone auf. Nun fährt der Transplantator mit der neuen Patrone in Pfeilrichtung (91) aus und schwenkt wieder in die 6-Uhr-Stellung zurück.

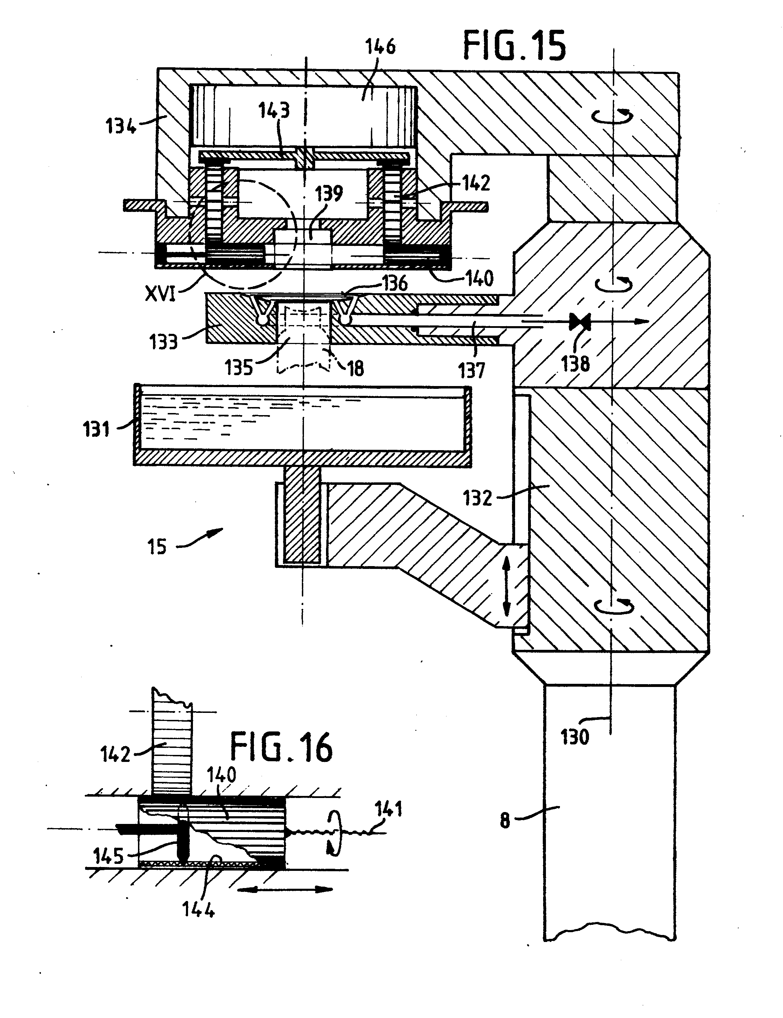

Schließlich zeigt Fig. 15 den oben am Hilfsträger (8) befestigten Manipulator (15). Er besteht aus drei um die gleiche vertikale Achse (130) schwenkbaren Komponenten, nämlich (von unten nach oben) einer mit physiologischer Kochsalzlösung gefüllten Sicherheitsschale (131), die an einer Nabe (132) auf und ab verfahrbar ist, einem Corneateller (133) und einem Transplantatgreifer (134). Eine vertikale zylindrische Bohrung (135) des Corneatellers (133) hat den gleichen Durchmesser wie die Bohrung des Bulbushalterings (14) und ist so angeordnet, daß der Trepan (18), der strichpunktiert angedeutet ist, in seiner 12-Uhr-Stellung von unten einfahren kann. Dabei muß selbstverständlich die Sicherheitsschale (131) weggeschwenkt sein. Der Bereich um die Bohrung (135) auf dem Corneateller ist als ringförmige Saugzone (136) zum Auflegen der Spendercornea ausgebildet. Die Saugzone ist der Corneaform entsprechend konkav gewölbt und hat zwei Ringschlitze, die über eine Saugleitung (137) und ein Ventil (138) an eine Vakuumquelle angeschlossen sind.

Der Transplantatgreifer hat bei entsprechender Winkelstellung koaxial über der Bohrung (135) des Corneatellers eine Aufnahmeöffnung (139) an seiner Unterseite. Diese ist umgeben von mehreren sternförmig angeordneten Greiferwalzen (140), welche zum gemeinsamen Mittelpunkt weisende Nadeln (141) tragen, die, wie aus dem Ausschnitt nach Fig. 16 ersichtlich, die Form eines feinen Korkenziehers haben. Die Greiferwalzen (140) haben eine Außenverzahnung und stehen in Eingriff mit Zwischenrädern (142), welche durch ein gemeinsames Sonnenrad (143) von einem Motor (146) angetrieben werden. Die Greiferwalzen (140) haben außerdem ein Innengewinde (144), in welches die Spitzen eines feststehenden T-Stücks (145) eingreifen. Wenn somit die Greiferwalzen angetrieben werden, machen sie, dem Gewinde (144) folgend, in ihrem Gehäuse eine translatorische Bewegung, derart, daß alle Nadeln (141) gleichzeitig zur Mitte wandern und sich in das Transplantat seitlich eindrehen bzw. zurückgeschraubt werden. Der Transplantatgreifer ist so bemessen, daß er durch Hochschieben der Sicherheitsschale (131) mit seinem unteren Teil, in welchem sich das aufgehängte Transplantat befindet, in die Konservierungsflüssigkeit eingetaucht werden kann.

Der Operationsverlauf stellt sich insgesamt wie folgt dar:

Vorbereitend muß das Zentrum der Empfängercornea markiert werden. Dies geschieht vorzugsweise in einer speziellen Sitzung, beispielsweise am Tage vor der Operation. Als Hilfsinstrument dient ein Spaltlampenvorsatz, in welchem der Arzt eine Schar konzentrischer Kreise und ein Fadenkreuz sieht. Diese Kreisschablone läßt sich konzentrisch auf die kreisförmige Übergangszone zwischen der durchsichtigen Cornea und dem undurchsichtigen Bindegewebe einstellen. Als Markierung des Corneazentrums kann unter örtlicher Anästhesie ein kleiner Farbpunkt o. dgl. angebracht werden.

Ferner kann vorbereitend das Transplantat gewonnen und bereitgestellt werden. Hierzu wird die Spendercornea mit dem Epithel nach unten auf den Corneateller (133) aufgelegt und mittels eines Spiegels oder des in die 12-Uhr-Stellung gefahrenen Achseinstellinstruments (17), d. h. auch unter Zuhilfenahme des Monitors (21), bezüglich der Bohrung (135) zentriert. Bei entsprechender Programmierung folgen nun die von der Vorrichtung selbsttätig ausgeführten Arbeitsgänge:

- das Vakuum der Saugzone (136) wird eingeschaltet;
- der Trepan (18) fährt in die 12-Uhr-Stellung und von unten in die Bohrung des Corneatellers (133) ein;
- das Trepanvakuum wird eingeschaltet;
- das Rohrmesser (52) läuft an und führt seine Schneidbewegung aus;
- der Trepan mit dem Transplantat fährt noch höher (Pfeilrichtung 50) in die Aufnahmeöffnung (139) des Transplantatgreifers hinein;

- dessen Motor läuft an und die Nadeln (141) drehen sich radial in die Schnittfläche des Transplantats ein;
- der Saugkopf des Trepans wird belüftet und der Trepan fährt nach unten aus der Bohrung des Corneatellers heraus und noch weiter nach unten;
- der Corneateller (133) wird weggeschwenkt;
- die Sicherheitsschale (131) wird eingeschwenkt und an gehoben, so daß das Transplantat in die Konservierungsflüssigkeit eintaucht und damit zunächst gesichert ist;
- die Transplantatvorbereitung wird am Monitor bestätigt und die Vorrichtung zur Operation freigegeben.

Zur unmittelbaren Vorbereitung der Operation wird der Patient auf der Liege (2) gelagert und sein Kopf mit Hilfe der Druckpolster (3) bzw. der Haltestifte möglichst sorgfältig und unverrückbar fixiert. Sodann folgt das Anästhesieren und das Anbringen der Haltefäden (41) (Fig. 4) am Bulbus. Die Vorrichtung wird jetzt in der Höhe und in der Horizontalen grob ausgerichtet, d. h. in den Pfeilrichtungen (7, 6 und 10). Dadurch kommt das in der 6-Uhr-Stellung befindliche Achseinstellinstrument (17) über das Auge des Patienten und grob in die Sehachse. Nun folgt die Feinausrichtung des Bulbus mittels der Haltefäden bei gleichzeitiger Feinjustierung des Achseinstellinstruments in der Horizontalen mit sehr viel geringerer Stellgeschwindigkeit, bis schließlich das Fadenkreuz, die Marke am Corneazentrum und die Makula am Monitor sichtbar zur Deckung gebracht sind.

Nach Drücken eines Programmknopfes schwenkt der Drehteller (11) so weit, daß der Trepan (18) in die 6-Uhr-Stellung kommt. Danach wird die Einstellbewegung (50) des Trepans (18) freigegeben. Der Operateur senkt den Trepan ab, wobei dieser zunächst in den Bulbushaltering (14) einfährt und schließlich diesen durchsetzend sich der Cornea nähert.

Der Operateur senkt den Trepan vorsichtig weiter ab bis zum Kontakt des Saugfußes (62) mit der Cornea des Patienten, was von der Seite her genau beobachtet werden kann. Nun wird der Programmknopf für die weiteren selbsttätigen Vorgänge gedrückt:

- die erreichte Höhenkoordinate wird gespeichert;
- der Bulbushaltering (14) senkt sich aufgrund dessen bezüglich des Hilfsträgers (8) genau auf die dem Trepan entsprechende Höhe ab, so daß auch die Anlagefläche (70) des Bulbushalterings genau aufliegt wie in Fig. 7 gezeigt;
- das Vakuum des Saugfußes (62) und des Saugschlitzes (71) wird eingeschaltet;
- der nächste Operationsschritt wird am Monitor angezeigt und kann vom Operateur mittels Programmtaste gestartet werden.

Der Operateur drückt wiederum den Programmknopf und startet damit die selbsttätige Trepanation und anschließende Transplantation im einzelnen wie folgt:

- Trepanation der Patientencornea;
- die Schnittiefe ist zuvor festeingestellt;
- der Trepan (18) mit daran hängender Patientencornea (78) fährt hoch;
- der Bulbushaltering bleibt unverändert;
- die Sicherheitsschale (131) wird abgesenkt und seitlich weggeschwenkt;
- der Drehteller (11) schwenkt den Transplantator (19) in die 12-Uhr-Stellung;
- der Transplantator fährt nach oben in die Aufnahmeöffnung (139) des Transplantatgreifers (134) ein und berührt das Transplantat;
- der innere Saugfuß (98) des Transplantators wird unter Vakuum gesetzt;

- der Transplantatgreifer (134) treibt die Greiferwalzen (140) mit den Nadeln (141) radial nach außen, so daß letztere das Transplantat freigeben;
- der Transplantator wird nach unten zurückgezogen, dreht in die 6-Uhr-Stellung und fährt in den Bulbushaltering (14) ein;
- der Transplantator wird auf die gespeicherte Höhenkoordinate abgesenkt, wodurch sich das Transplantat (112) genau in die Empfängercornea einpaßt;
- das Vakuum des äußeren Saugrings (99) des Transplantators wird eingeschaltet und damit auch der Trepanationsrand fixiert;
- die in der Klammerpatrone (95) enthaltenen acht Klammern (113) werden durch Einschalten des Schlagantriebs in Pfeilrichtung (108) (Fig. 10) eingesetzt;
- das gesamte Vakuum des Transplantators wird abgeschaltet (beide getrennten Saugleitungen 100 und 101).

Damit ist die Operation an sich abgeschlossen. Sind noch weitere Klammern erforderlich, so folgen noch zwei Klammerungsvorgänge wie folgt:

- der Transplantator (19) fährt hoch aus dem Bulbushaltering heraus;
- der Transplantator schwenkt in die 9-Uhr-Stellung und fährt in den Klammerpatronen-Wechsler (16) ein;
- dort wird die Klammerpatrone (95) abgenommen und eine Ersatzpatrone in einer um 15° geänderten Winkelstellung angesetzt;
- der Transplantator (19) fährt zurück und schwenkt erneut in die 6-Uhr-Stellung;
- er fährt in den Bulbushaltering (14) ein und senkt sich erneut bis zur Berührung mit dem Transplantat und der Empfängercornea ab. Das Vakuum beider Saugzonen wird wieder eingeschaltet;

- der zweite Klammerkranz in um 15° versetzter Winkelstellung wird eingesetzt;
- es folgt sodann der dritte Klammerungsvorgang in identischer Einzelreihenfolge;
- das Vakuum des Bulbushalterings (14) wird abgeschaltet;
- der Turmkopf (4) mit allen von ihm getragenen Komponenten einschließlich des Bulbushalteringes fährt hoch und schwenkt um 90° seitlich weg;
- Anzeige "Ende der Operation" im Monitor.

1 Grundgestell
2 Patientenliege
3 Druckpolster
4 Turmkopf
5 Turmachse
6 Drehbewegung
7 Hubbewegung
8 Hilfsträger
9 Schieberohr
10 Translation
11 Drehteller
12 Drehtellerachse
13 Schwenkbewegung um 12
14 Bulbushaltering
15 Manipulator
16 Klammerpatronen-Wechsler
17 Achseinstellinstrument
18 Trepan
19 Transplantator
20 Steuerkonsole
21 Monitor
28 Linsensystem
29 Linsensystem
30 Hauptstrahlengang
31 halbdurchlässiger Spiegel
32 Spiegel
33 Beleuchtungsquelle
34 Makula
35 halbdurchlässiger Spiegel
36 halbdurchlässiger Spiegel
37 Parallelstrahlung
38 Cornea
39 Markierung
40 Fadenkreuz
41 Haltefaden
42 Videokamera
50 Einstellbewegung
51 Rohrmesserachse
52 Rohrmesser
53 Verdickung
54 Rändelmutter
55 Hohlwelle
56 Rotor
57 Lager
58 Stator
59 Schneidzustelung
60 Halter
61 Schaft
62 Saugfuß
63 Schaftabschnitt
64 Saugleitung
65 Ventil
66 Arm
67 Schlitten
68 Absenkbewegung
69 Achse, fluchtend mit 51
70 Anlagefläche
71 Saugschlitz
72 Bohrung
73 Sammelkanal
74 Saugleitung
75 Ventil
76 Saugbohrung
77 Anlagefläche
78 Cornea

79 Feuchtigkeitssensor
80 Rohrmesserabschnitt
81 Zacken
82 Schneidfacette
83 Schneidrichtung
90 Halter
91 Verstellbewegung
92 Schaft
93 Hülse von 90
94 Schlaghülse
95 Klammerpatrone
96 Bajonettverschluß
97 Ansatz
98 Saugfuß
99 Saugring
100 Saugleitung
101 Saugleitung
102 Ventil
103 Ventil
104 Stempel
105 Endabschnitt von 104
106 Druckfeder
107 Antriebsring
108 Schlagrichtung
109 Feuchtigkeitssensor
110 Saugkanal
111 Klammerkanal
112 Transplantator
113 Klammer
113a Klammer
113b Klammer
114 Stirnfläche
120 Schacht
121 Magazinpackung
122 Führungsstreifen
123 Drehfutter
124 Drehfutterachse
125 Schachtöffnung
130 Schwenkachse
131 Sicherheitsschale
132 Nabe
133 Corneateller
134 Transplantatgreifer
135 Bohrung
136 Saugzone
137 Saugleitung
138 Ventil
139 Aufnahmeöffnung
140 Greiferwalze
141 Nadel, gewendelt
142 Zwischenrad
143 Sonnenrad
144 Innengewinde
145 T-Stück
146 Motor
147 Prisma
148 Lichtleiter
149 Faserbündel

Ansprüche:

1. Vorrichtung zur Transplantation der Cornea des menschlichen Auges mit einem als Patientenauflage ausgebildeten Grundgestell (1), einer Kopfauflage mit Fixiervorrichtung (3) und einem während der Operation fixierbaren Bulbushaltering (14), der mit seiner saugfähigen unterseitigen Oberfläche (70) im corneal-skleralen Übergangsbereich auf das Auge auflegbar ist, gekennzeichnet durch folgende Merkmale:

- An dem Grundgestell (1) ist ein mittels elektronisch steuerbarer Antriebe dreidimensional verstellbarer Träger (11) gelagert;
- An dem Träger (11) sind wenigstens drei Instrumente angeordnet, nämlich ein optisches Achseinstellinstrument (17) zur gleichzeitigen monokularen Beobachtung der Corneaoberfläche und des Fundus des Patientenauges, ein Trepan (18) zum Herausschneiden eines kreisscheibenförmigen zentralen Teils der Cornea und ein Transplantator (19) zur Übertragung eines Cornea-Transplantats (112) und zu dessen Fixierung im Patientenauge;
- Die Positionsdaten der Instrumente sind speicherbar und es ist eine elektronische Steuereinrichtung vorgesehen, mit deren Hilfe der Träger (11) und die Instrumente so verstellt werden können, daß ein auszuwählendes Instrument auf Befehl sich in die Position bewegt, die zuvor ein anderes Instrument einnahm.

2. Vorrichtung nach Anspruch 1, dadurch gekennzeichnet, daß der Träger ein um eine horizontale Achse (12) drehbar gelagerter Drehteller (11) ist, an dem die Instrumente sternförmig mit radialen Mittelachsen angeordnet sind.

3. Vorrichtung nach Anspruch 2, dadurch gekennzeichnet, daß der Drehteller (11) in einem Hilfsträger (8) gelagert ist, der zusammen mit dem Drehteller (11) in Richtung der Achse (12) horizontal verschiebbar (10), um eine vertikale Achse (5) drehbar (6) und in Richtung dieser vertikalen Achse (5) höhenverschiebbar (7) ist.

4. Vorrichtung nach Anspruch 2, dadurch gekennzeichnet, daß der Bulbushaltering (14) in vertikaler Richtung verschiebbar (68) und genau einstellbar an dem Hilfsträger (8) angebracht ist.

5. Vorrichtung nach Anspruch 4, dadurch gekennzeichnet, daß der Bulbushaltering (14) aus einem durchsichtigen Werkstoff gefertigt ist und/oder optische Fasern (148, 149) zur Beleuchtung und Beobachtung des Ringinnenraums enthält.

6. Vorrichtung nach Anspruch 3, dadurch gekennzeichnet, daß an dem Hilfsträger (8) ein Manipulator (15) angebracht ist zum Halten der Spendercornea und des Transplantats (112) und zum Übertragen des Transplantats auf den Transplantator (19).

7. Vorrichtung nach Anspruch 3, dadurch gekennzeichnet, daß an dem Hilfsträger (8) ein selbsttätiger Klammerpatronen-Wechsler (16) angebracht ist zum Entfernen einer leeren Klammerpatrone (95) von einem Klammerapparat des Transplantators (19) und zum Ansetzen einer neuen Klammerpatrone.

8. Vorrichtung nach Anspruch 1, dadurch gekennzeichnet, daß das Achseinstellinstrument (17) als monokulares Mikroskop mit Beleuchtung (33) zur Betrachtung des Augenhintergrundes ausgebildet ist, daß es eine Markierung (40) in Sehfeldmitte aufweist und daß vor und hinter dem Linsensystem (28) halbdurchlässige Spiegel (35, 36) angeordnet sind, mittels derer ein Teil der Strahlung abgezweigt und dem Hauptstrahlengang (30) wieder zugefügt wird, wobei dieser zweite Strahlengang (37) durch ein zweites Linsensystem (29) verläuft.

9. Vorrichtung nach Anspruch 8, dadurch gekennzeichnet, daß am Achseinstellinstrument (17) eine Videokamera (42) angeordnet ist, welche das empfangene Bild auf einen Monitor (21) überträgt.

10. Vorrichtung nach Anspruch 1, dadurch gekennzeichnet, daß der Trepan (18) ein Rohrmesser (52) und einen bezüglich des Trägers (11) in Achsrichtung des Rohrmessers verschiebbaren (50) und genau einstellbaren Halter (60) aufweist, daß an dem Halter ein Schaft (61) und an dessen Ende ein Saugfuß (62) in Gestalt eines gasdurchlässig ausgebildeten Formkörpers befestigt ist, der durch eine Längsbohrung (64) des Schafts hindurch an ein Vakuum angeschlossen ist und eine der äußeren Oberflächenform der menschlichen Cornea angenäherte Anlagefläche (77) aufweist, daß das Rohrmesser (52) den Schaft (61) und den Saugfuß (62) umfassend drehbar gelagert und in Achsrichtung des Rohrmessers bezüglich des Halters (60) verschiebbar (59) und genau einstellbar ist und daß das Rohrmesser (52) in den Bulbushaltering (14) passend einfügbar ist.

11. Vorrichtung nach Anspruch 10, dadurch gekennzeichnet, daß die axiale Beweglichkeit des Rohrmessers (52) zwischen einer hinter die Anlagefläche (77) des Saugnapfs (62) zurückgezogenen Bereitschaftsstellung und einer voreinstellbaren extremen Schneidstellung begrenzt ist.

12. Vorrichtung nach Anspruch 10, dadurch gekennzeichnet, daß das Rohrmesser (52) mittels eines Motors angetrieben wird.

13. Vorrichtung nach Anspruch 10, dadurch gekennzeichnet, daß das Rohrmesser (52) an seiner Schneidkante Zacken (81) mit angeschliffenen Schneidfacetten (82) hat, die bei der Drehbewegung vorauslaufen.

14. Vorrichtung nach Anspruch 10, dadurch gekennzeichnet, daß der Saugfuß (62) einen feuchtigkeitsempfindlichen Sensor (79) aufweist, der das angelegte Vakuum verringert und ein Signal auslöst.

15. Vorrichtung nach Anspruch 1, dadurch gekennzeichnet, daß der Transplantator (19) einen länglichen Schaft (92) und einen mit diesem fest verbundenen Halter (90) aufweist, der bezüglich des Trägers (11) und in Richtung der Achse des Schafts (92) verschiebbar (91) und genau einstellbar ist, daß am Ende des Schaftes ein Saugfuß (98, 99) angebracht ist, der durch Längsbohrungen (100, 101) des Schaftes hindurch an ein Vakuum angeschlossen ist und der äußeren Oberflächenform der menschlichen Cornea angenäherte Anlageflächen aufweist, und daß der Saugfuß wenigstens einen in die Anlagefläche mündenden Klammerkanal (111) sowie einen Stempel (104) aufweist, um eine in dem Klammerkanal enthaltene Klammer (113) auszustoßen, wobei der Saugfuß (98, 99) in den Bulbushaltering (14) passend einfügbar ist.

16. Vorrichtung nach Anspruch 15, dadurch gekennzeichnet, daß U-förmige Klammern (113) vorgesehen sind mit einem Verbindungssteg und zwei parallelen Schenkeln, die nach innen weisende Widerhaken tragen.

17. Vorrichtung nach Anspruch 15, dadurch gekennzeichnet, daß V-förmige Klammern (113a) vorgesehen sind, deren erster Schenkel gestreckt und zugespitzt und deren zweiter Schenkel der Wölbung der Corneaoberfläche entsprechend nach außen gekrümmt ist und am Ende einen Widerhaken trägt.

18. Vorrichtung nach Anspruch 15, dadurch gekennzeichnet, daß V-förmige Klammern (113b) vorgesehen sind, deren erster Schenkel kreisförmig nach außen gekrümmt und zugespitzt und deren zweiter Schenkel der Wölbung der Corneaoberfläche entsprechend nach außen gekrümmt ist und am Ende einen Widerhaken trägt.

19. Vorrichtung nach Anspruch 15, dadurch gekennzeichnet, daß die Anlagefläche des Saugfußes eine äußere Ringzone (99) und eine Innenzone (98) aufweist, die über getrennte Leitungen (101 und 100) an einzeln einstellbare Vakuumquellen angeschlossen sind.

20. Vorrichtung nach Anspruch 19, dadurch gekennzeichnet, daß mehrere Klammerkanäle (111) vorgesehen sind, deren Mündungsöffnungen im Bereich zwischen der Ringzone (99) und der Innenzone (98) kreisförmig mit gleichen Abständen und radialer Längserstreckung angeordnet sind.

21. Vorrichtung nach Anspruch 20, dadurch gekennzeichnet, daß die Klammerkanäle (111) in Schaftlängsrichtung verlaufen und die überstehenden Stempel (104) mittels eines Schlagorgans (107, 94) gemeinsam betätigbar sind.

22. Vorrichtung nach Anspruch 21, dadurch gekennzeichnet, daß der Fußteil des Schaftes (92), der Saugfuß (98, 99) und die Stempel (104) eine Klammerpatrone (95) bilden, die mittels einer Schnellkupplung (96) leicht von der Hülse (93) abnehmbar und wahlweise in unterschiedlichen Winkelstellungen ansetzbar ist, wobei die Schlagkraft mittels einer längsbeweglichen Schlaghülse (94) auf die Stempel übertragen wird.

23. Vorrichtung nach Anspruch 7, dadurch gekennzeichnet, daß der Klammerpatronen-Wechsler (16) in einem Magazinschacht (120) mehrere mit Klammern geladene Klammerpatronen enthält und diese der Reihe nach in unterschiedlicher Winkelorientierung an die Hülse (93) des Transplantators (19) ansetzt.

24. Vorrichtung nach Anspruch 23, dadurch gekennzeichnet, daß die sterilen Klammerpatronen (95) in unterschiedlicher Winkelorientierung in einer Magazinpackung (121) enthalten sind, welche in dem Klammerpatronen-Wechsler (16) eingefügt werden kann.

25. Vorrichtung nach Anspruch 23, dadurch gekennzeichnet, daß koaxial zu dem in den Wechsler eingefahrenen Transplantator (19) ein drehbarer und längsverschiebbarer Greifer (123) angeordnet ist, welcher die Klammerpatronen (95) erfaßt und dreht und daß diese dem Greifer selbsttätig zugefördert bzw. nach Gebrauch ausgeworfen werden.

26. Vorrichtung nach Anspruch 6, dadurch gekennzeichnet, daß der Manipulator (15) einen Transplantatgreifer (134), einen horizontal angeordneten Corneateller (133) und eine Sicherheitsschale (131) aufweist, welche um eine gemeinsame vertikale Achse (130) drehbar gelagert sind, wobei die eine Konservierungsflüssigkeit enthaltende Sicherheitsschale (131) außerdem in Höhenrichtung verfahrbar ist, so daß der Transplantatgreifer (134) teilweise in die Flüssigkeit eintauchen kann.

27. Vorrichtung nach Anspruch 26, dadurch gekennzeichnet, daß der Corneateller (133) eine dem Außendurchmesser des Trepans (18) entsprechende zentrale Öffnung (135) und eine diese umgebende ringförmige Anlagefläche (136) aufweist, welche eine der Oberflächenform der corneanahen Sklerateile des menschlichen Bulbus oculi angenäherte konkave Form hat, als gasdurchlässige Saugzone ausgebildet und an ein Vakuum angeschlossen ist.

28. Vorrichtung nach Anspruch 27, dadurch gekennzeichnet, daß der Transplantatgreifer (134) mehrere sternförmig angeordnete und mit ihren Spitzen zum Sternmittelpunkt weisende nadelähnliche Tragelemente (141) aufweist, die sich mit Hilfe einer entsprechenden Antriebsanordnung (140 bis 143) gemeinsam radial bewegen können und in das Transplantat (112) an seiner Schnittfläche eindringen.

0208950

FIG.1

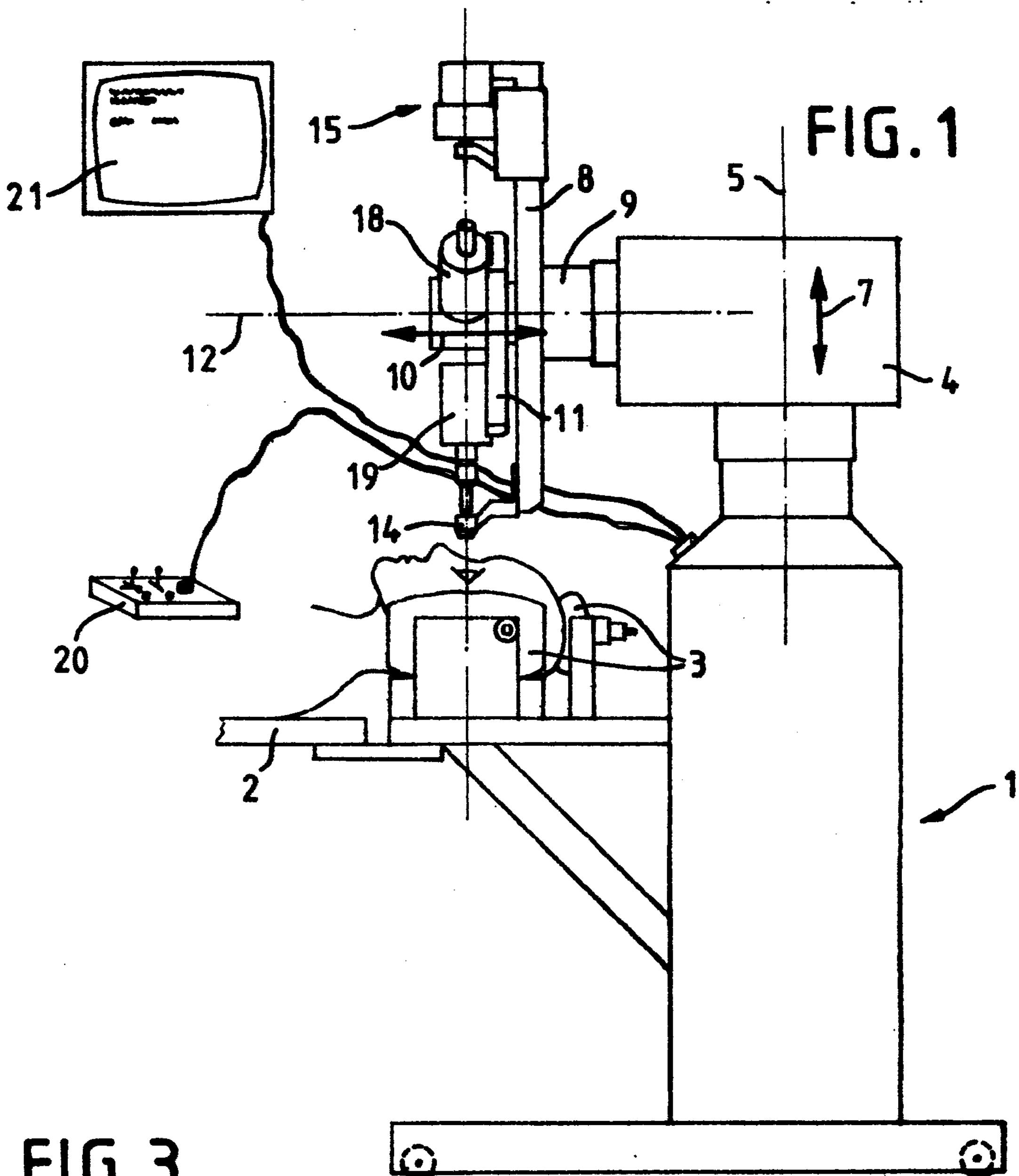


FIG.3

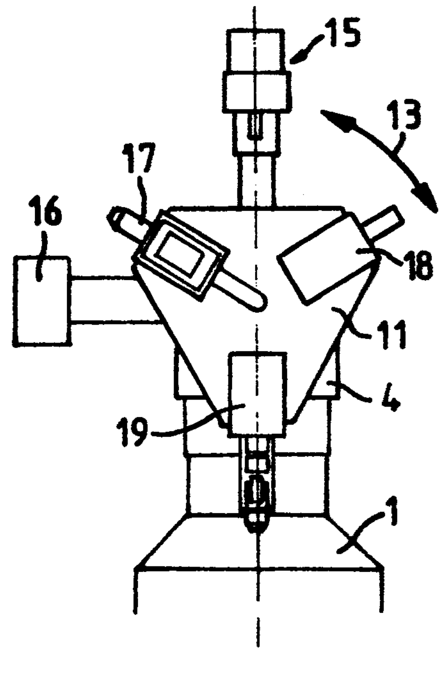


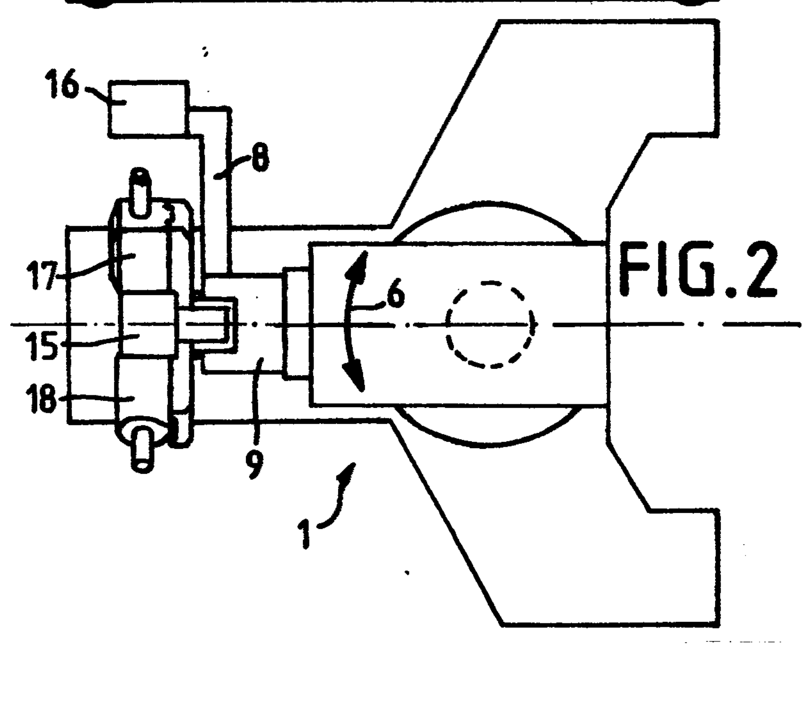


FIG.2

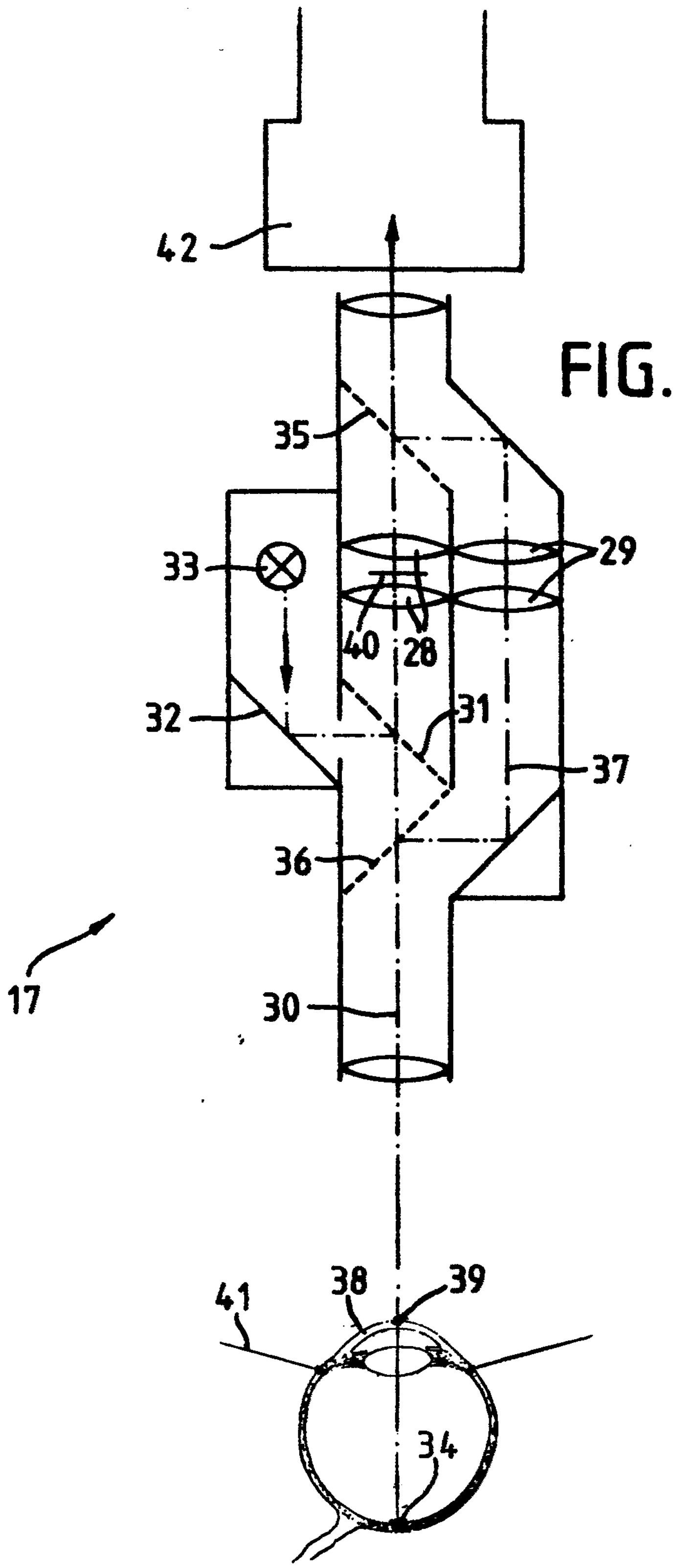


FIG. 4

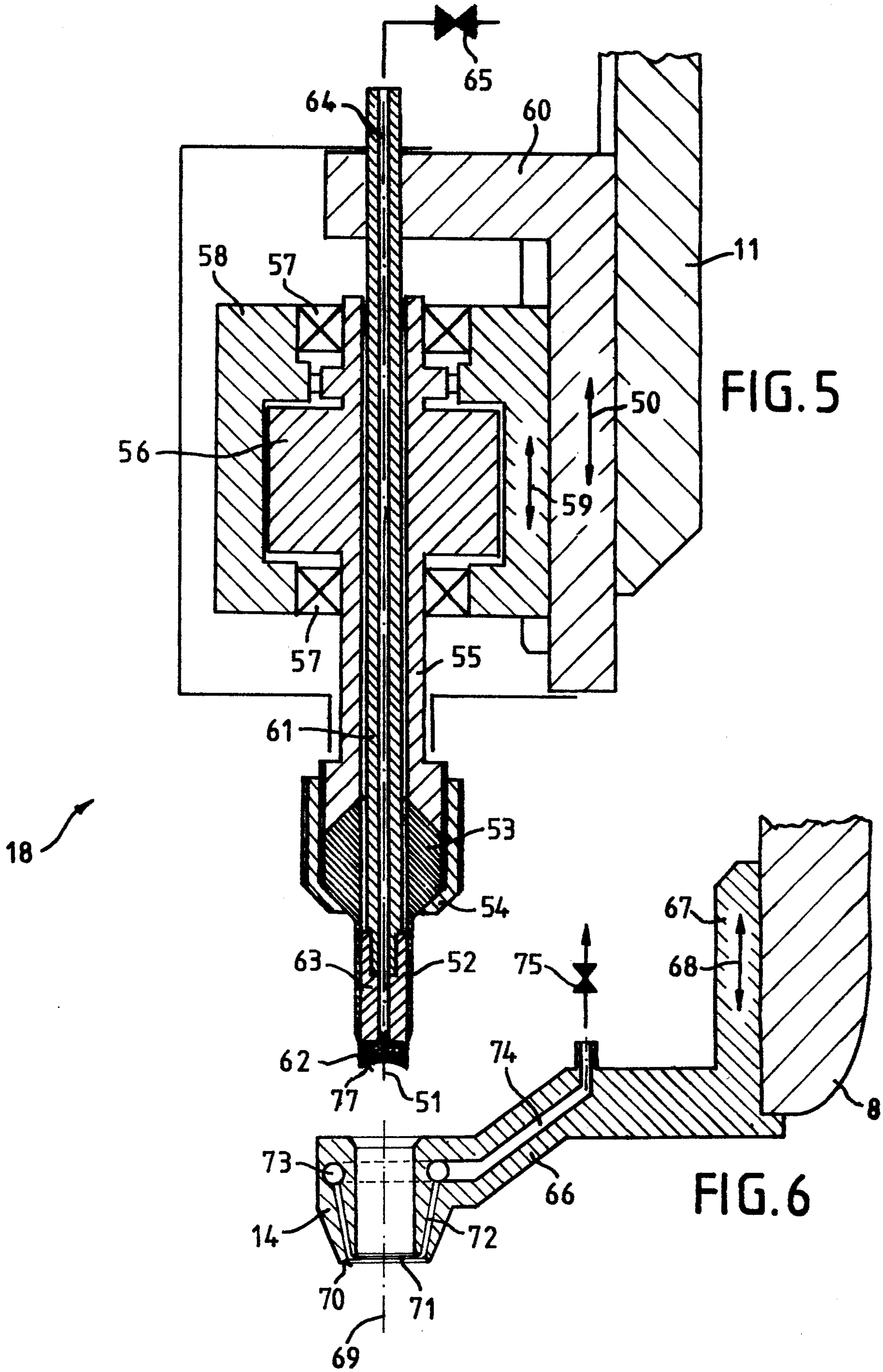
65
64
60
11
58
57
56
50
59
57
55
61
18
53
54
67
63
52
75
68
62
74
77
51
8
73
66
14
72
70
71
69

FIG. 5

FIG. 6

FIG. 7

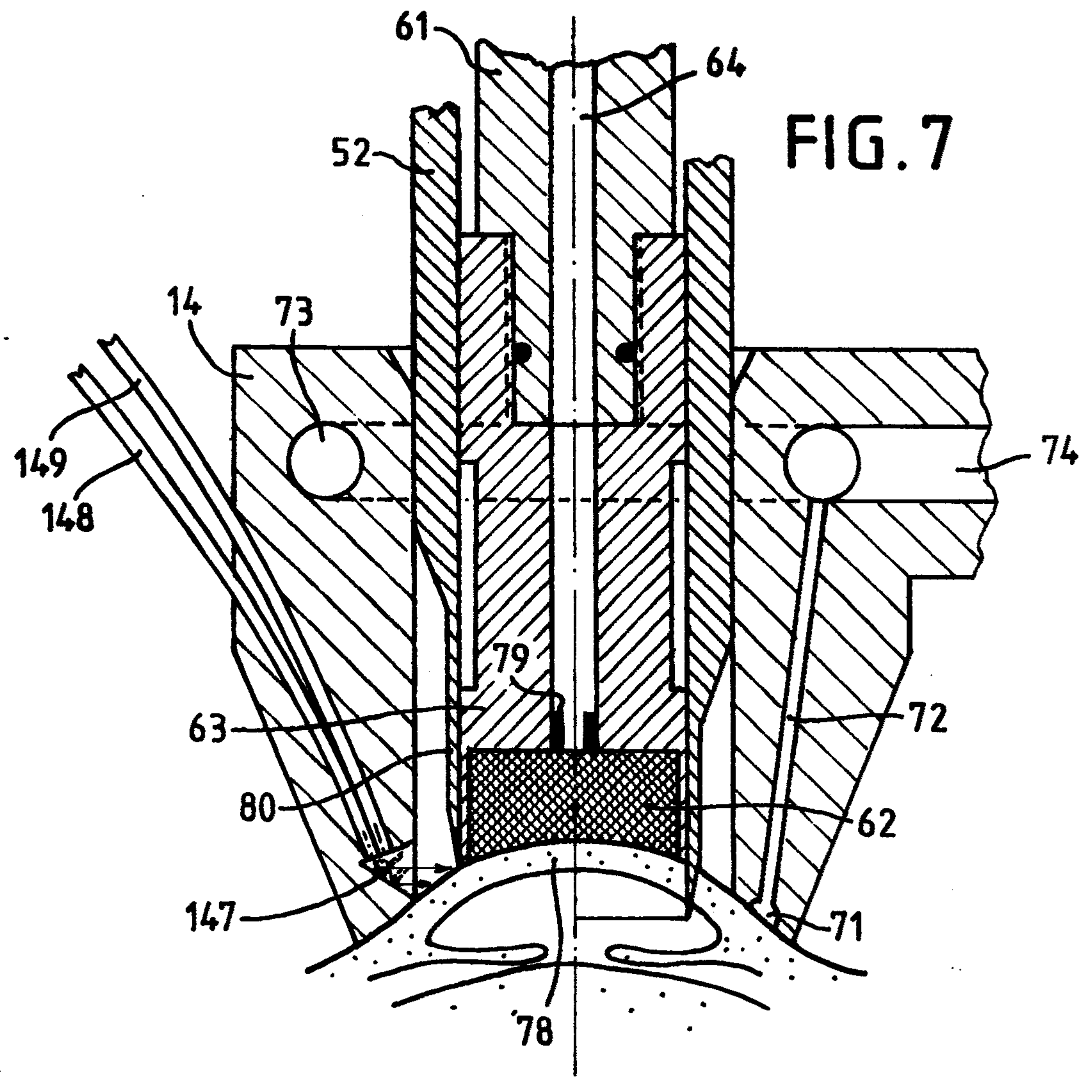


FIG. 8

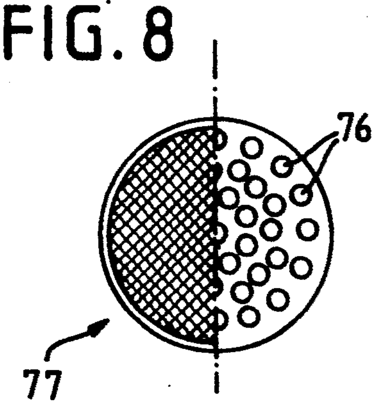


FIG. 9

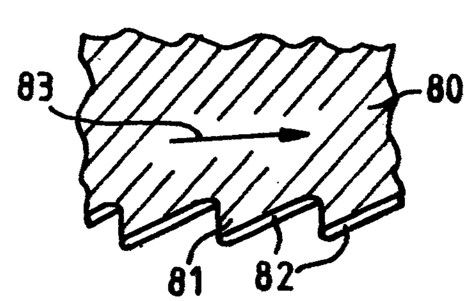

FIG.10

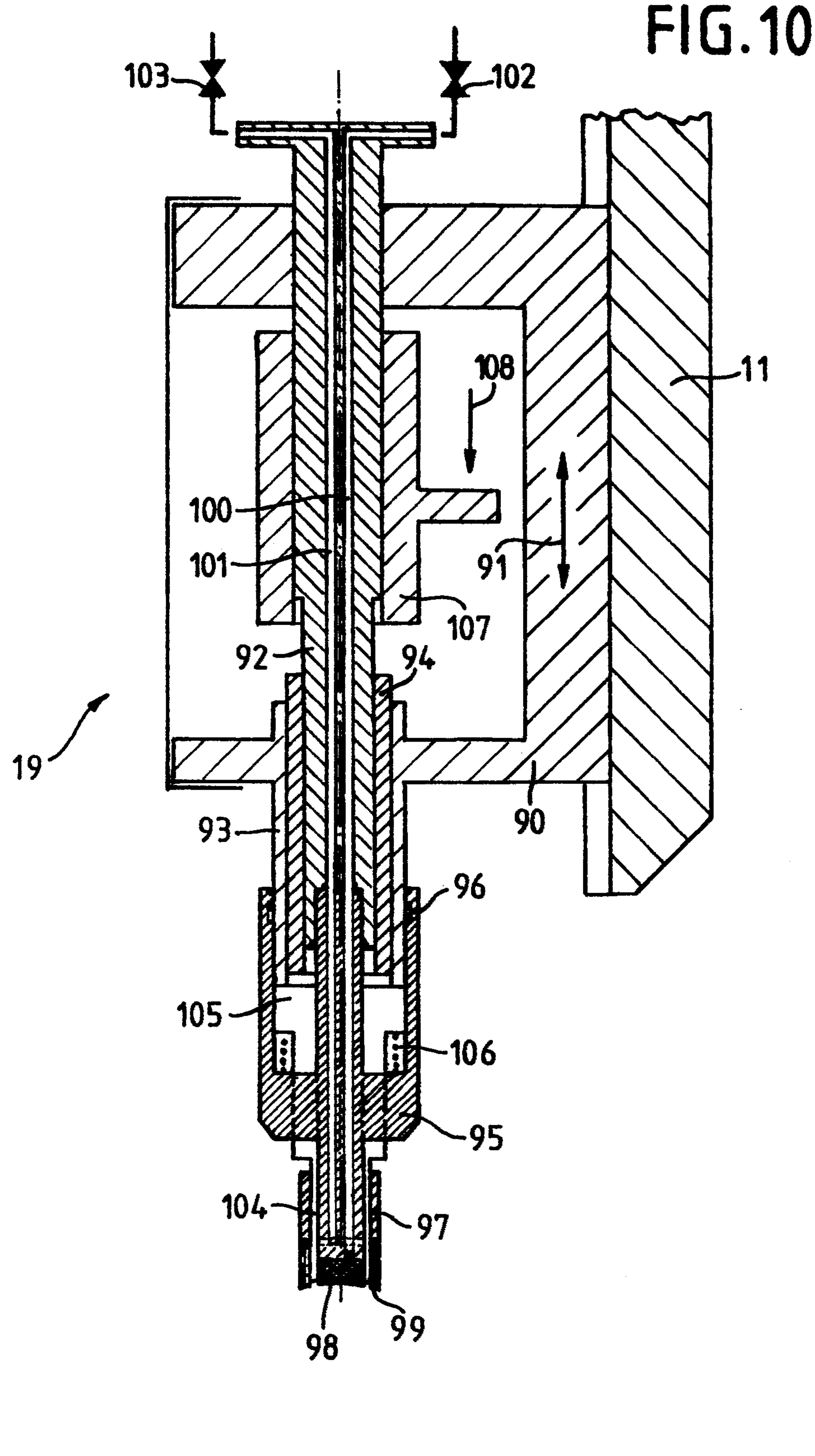
103
102
11
108
100
101
91
107
92
94
19
93
90
96
105
106
95
104
97
98
99

FIG.12

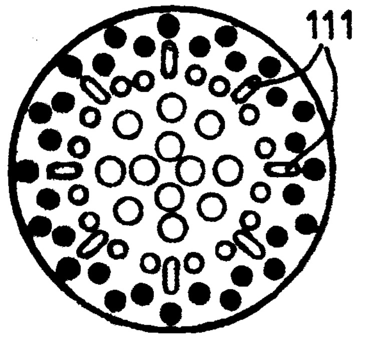


FIG.11

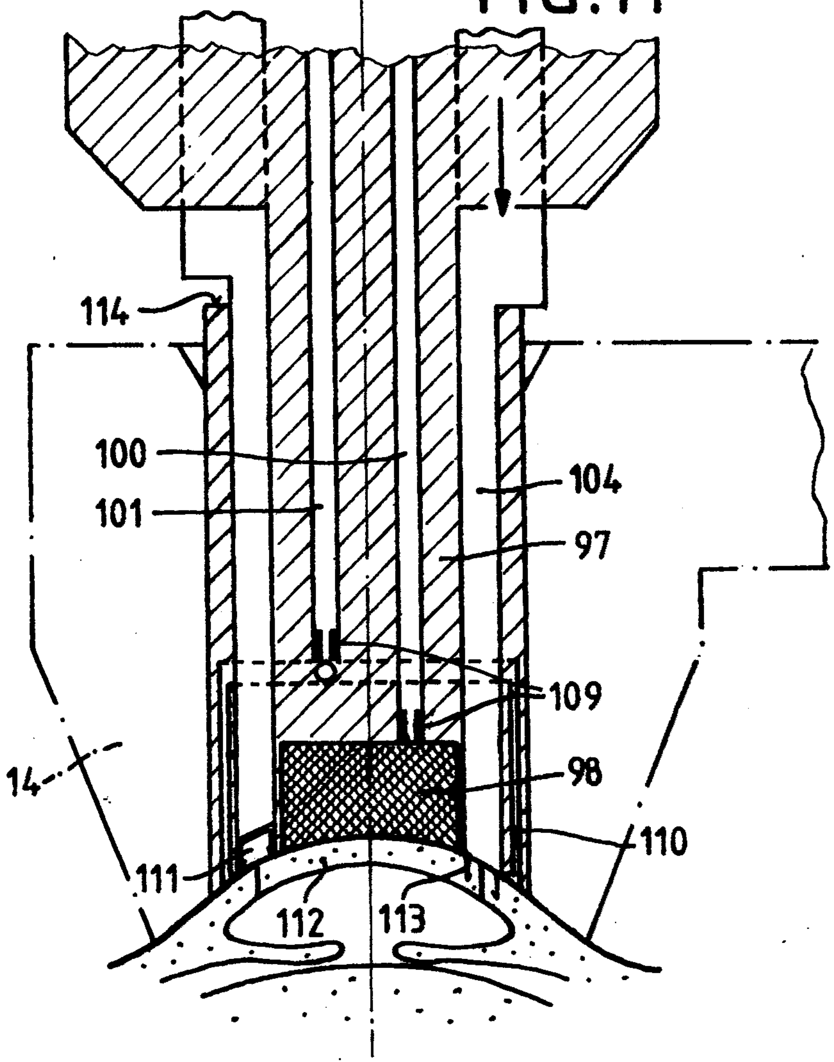


FIG.13

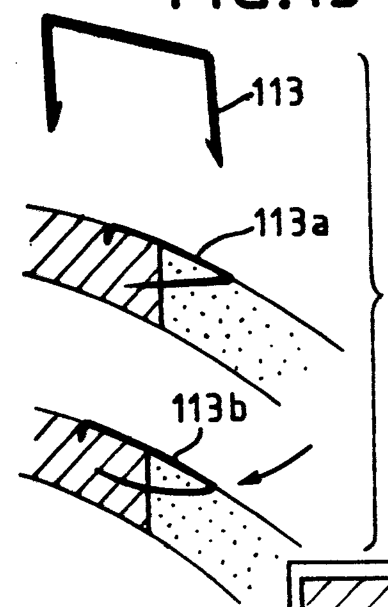


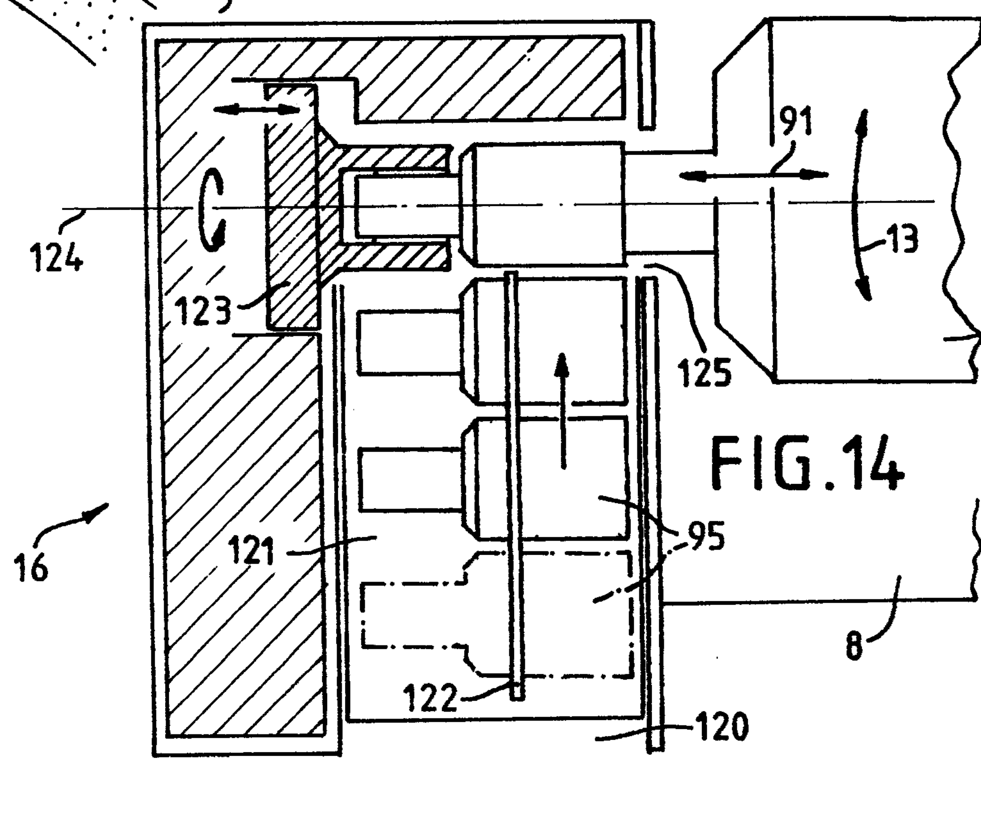


FIG.14

0208950

FIG. 15

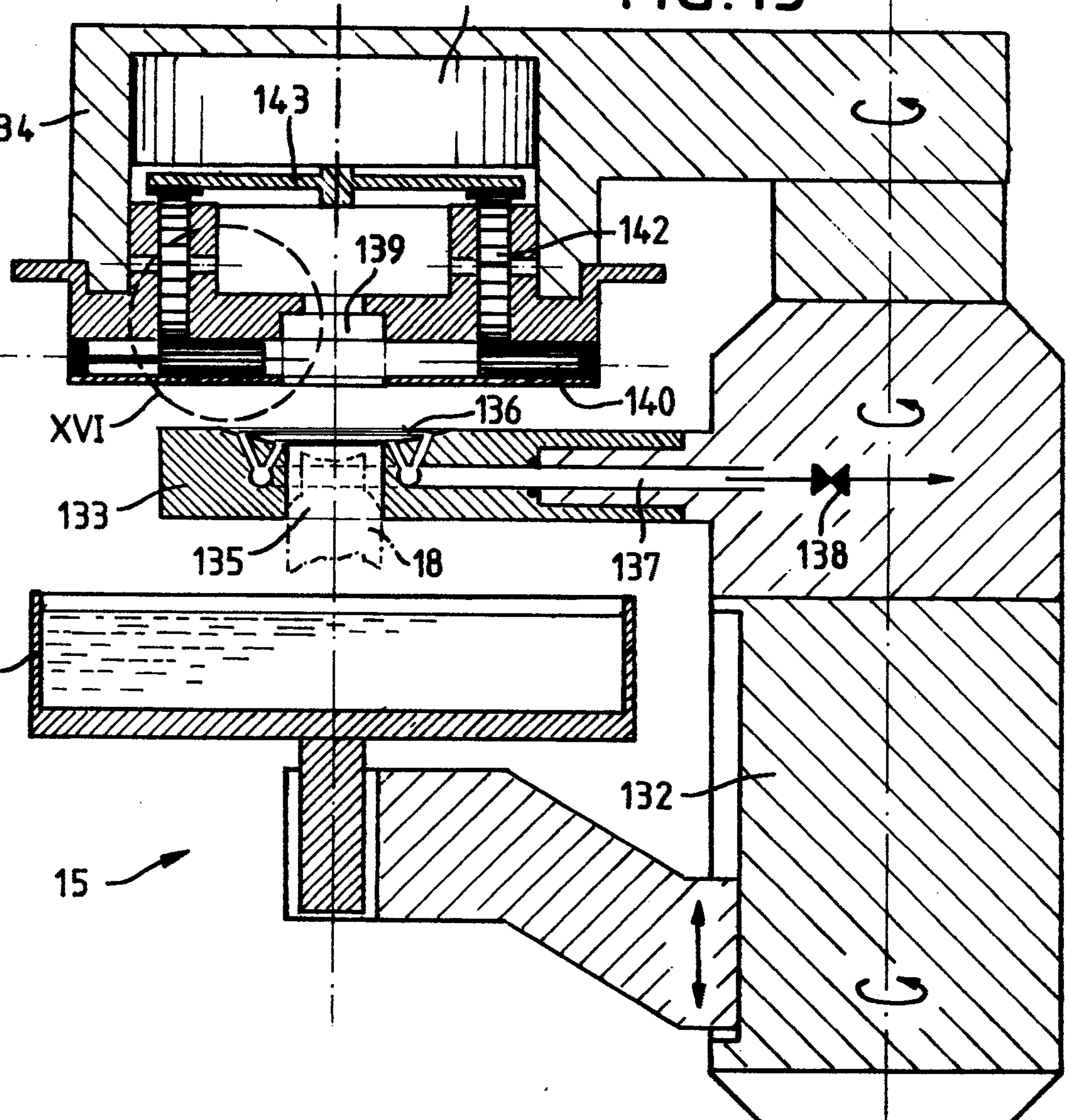
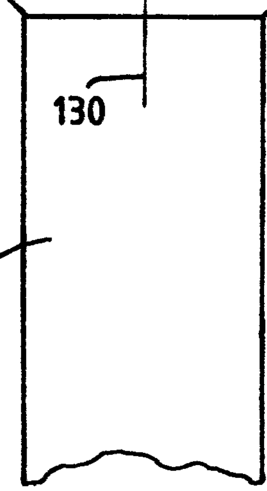


FIG. 16